# EUROPEAN PATENT APPLICATION

(11) **EP 2 489 428 A1**
(43) Date of publication of application: **22.08.2012**
(21) Application number: 11001419.8
(22) Date of filing: 21.02.2011
(51) Int. Cl.: B01J 2/02, B01J 2/04, B29B 11/06, B29C 51/00, C07C 67/08, C07C 67/52, C08K 5/10

(54) **Process and device for the preparation of an ester in granule form**

(71) Applicant: Emery Oleochemicals GmbH, 40589 Düsseldorf (DE)
(72) Inventor: Daute, Peter, 27616 Beverstedt (DE); Reiners, Wilhelm, 27612 Loxstedt (DE); Schäfer, Martin, 27616 Stubben (DE); Frerichs, Udo, 27612 Loxstedt (DE); Hildebrandt, Hinrich, 21769 Armstorf (DE); Ellerbrake, Joern, 27570 Bremerhaven (DE)
(74) Representative: Kinkeldey, Daniela

(57) **Abstract**

The present invention relates to a process for the preparation of an ester comprising an after-treatment of the ester to give an ester in granule form of defined particle size distribution, a device for carrying out this process, a process for the preparation of a thermoplastic composition comprising the ester prepared according to the invention, a process for the production of a shaped article comprising the ester according to the invention or the thermoplastic composition according to the invention, a process for the production of a packed product, a process for the production of an at least partly coated object, and uses of the esters according to the invention as an additive in various compositions.

## Description

The present invention relates to a process for the preparation of an ester comprising an after-treatment of the ester to give an ester in granule form of defined particle size distribution, a device for carrying out this process, a process for the preparation of a thermoplastic composition comprising the ester prepared according to the invention, a process for the production of a shaped article comprising the ester according to the invention or the thermoplastic composition according to the invention, a process for the production of a packed product, a process for the production of an at least partly coated object, and uses of the esters according to the invention as an additive in various compositions.

Esters, in particular those based on aliphatic carboxylic acids and alcohols, are employed successfully in a large number of uses. In the awareness that raw materials from fossil deposits are becoming scarcer, new sources of raw materials are being sought. Oils from animal or plant renewable raw materials which are broken down to fatty acids e.g. by ozonolysis and refunctionalized or derivatized in further steps appear to be particularly promising.

Ester preparation is an industrially important derivatization, for which various processes are known. EP 0 342 357 A2 describes a device and a process for carrying out esterifications. In this, esters are prepared from alcohols and fatty acids in a production plant at 200 to 250 °C, the reaction mixture being led continuously over a particularly hot reaction zone with a short contact time and the preparation being carried out over reaction times of up to 20 hours.

With respect to industrial esterification reactions, there is need for improvement in various aspects in order to meet the requirements and demands of the market. The known processes have at least one, as a rule several of the disadvantages outlined below:
- coloured nature or inadequate colourlessness of the products
- undesirable by-products,
- inadequate stability
- low efficiency, high energy consumption, high production costs,
- low yield,
- impurities, in particular traces of heavy metals,
- long reaction times.

In particular, in the case of solid additives or mixtures of solid additives there is the demand for a particulate make-up, for example for simple metering or to avoid demixing of the components during transportation. Furthermore, a chemical composition which is as uniform and constant as possible over a long production time is desirable. This ensures optimization and where appropriate automation of processing processes.

The present invention was based on the object of at least partly overcoming the disadvantages emerging from the prior art.

In particular, the present invention was based on the object of providing a process and a device for providing particulate esters in granule form which have a certain, uniform particle size distribution during a production over a long period of time and therefore lead only to small amounts of product lying outside a specification.

In particular, the present invention was based on the object of providing a process for producing particulate esters in granule form, wherein the formation of angel hairing (thin "hairy" fibres of the solid ester) as a by product is avoided. Angel hairing is a problem in granule handling since it easily charges electrostatically and, thus, sticks to the walls of tubings, reactors and product containers. Besides, it may also promote blocking of valves, sluices and other apparatuses.

In particular, the present invention was based on the object of providing a process for producing particulate esters in granule form, wherein the formation of agglomerates is avoided. An agglomerate in the context of the production of granules is referred to a plurality of granules sticking together. This may occur when particles of the ester come into contact at temperatures, which are close to the melting point of the ester. The formation of agglomerates implies difficulties with regard to uniformity and distribution of particle size, metering, demixing during transport etc.

A further object was to provide esters in granule form which have the lowest possible content of coarse particles (particle size ≥ 2 mm), which are not very advantageous for thermoplastic moulding compositions, and of fine particles (particle size ≤ 50 µm), which are unfavourable for work safety reasons.

A contribution towards achieving at least one of the abovementioned objects is made by the subject matter of the category-forming claims, the sub-claims dependent upon these representing further embodiments according to the invention.

The present invention provides a process for the preparation of an ester at least based on
a. at least one alcohol component,
b. at least one carboxylic acid component,
as process components in a reactor, comprising the process steps:
i. provision of the process components,
ii. reaction of the process components to give an ester A,
iii. after-treatment of the ester A to give an ester in granule form,
wherein the after-treatment comprises at least a process for the preparation of an ester in granule form which can be carried out in a spray tower and comprises at least the after-treatment steps:
aa. provision of the ester A as a fluid stream,
bb. optionally addition of auxiliary substances,
cc. charging of the fluid stream with pressure,
dd. release and division of the fluid stream via a pressure release device into a contact region to give a discontinuous fluid stream and
ee. cooling of the discontinuous fluid stream in the contact region by a cooling fluid in counter-current, to give a solid particle stream,
wherein, according to formula (I), the variable *I₁* is in a range of from 0.05 to 4.0, ${I}_{1} = \left|\frac{{p}_{E}}{{T}_{2 , CF} - {T}_{1 , CF}}\right|$
where
p_{E} = pressure of the fluid stream before the pressure release device;
T_{1,CF} = temperature of the cooling fluid on entry into the contact region;
T_{2,CF} = temperature of the cooling fluid on exit from the contact region;
or
wherein, according to formula (II), the variable *I₂* is in a range of from 0.04 to 3.0, ${I}_{2} = \left|\frac{{T}_{1 , E} - {T}_{m , E}}{{T}_{2 , CF} - {T}_{1 , CF}}\right|$
where
T_{m,E} = melting point of the ester A;
T_{1,E} = temperature of the ester A as the fluid stream before the pressure release device;
T_{1,CF} = temperature of the cooling fluid on entry into the contact region;
T_{2,CF} = temperature of the cooling fluid on exit from the contact region;
or
wherein, according to formula (III), the variable *I₃* is in a range of from 0.04 to 0.95, ${I}_{3} = \frac{{p}_{E}}{{η}_{E}}$
where
p_{E} = pressure of the fluid stream before the pressure release device, determined in [bar];
η_{E} = viscosity of the fluid stream before the pressure release device, determined in [mPa·s],
or
a combination of two or more of these.

An after-treatment taking into account a combination of all three conditions is particularly preferred.

In principle, any alcohol component with one or more hydroxyl groups which is known to the person skilled in the art and appears to be suitable for carrying out the process according to the invention is suitable as the alcohol component for carrying out the process according to the invention. The term "alcohol component" as used here includes the alcohol in its protonated form, the alcohol in its deprotonated form, in particular salts of the alcohol, and also mixtures of the alcohol in its protonated form and its deprotonated form or mixtures of the alcohol in its protonated form, its deprotonated form and one or more salts of the alcohol.

Alcohols with a number of hydroxyl groups in a range of from 1 to 9, particularly preferably 3 to 8 and most preferably 3 to 6 are preferably employed as the alcohol component with one or more hydroxyl groups. The number of carbon atoms in the alcohol with one or more hydroxyl groups is preferably in a range of from 3 to 30, particularly preferably 3 to 18, furthermore preferably 3 to 10 and most preferably 3, 4, 6 or 8.

A technical grade alcohol can also be employed as the alcohol component. "Technical grade" in connection with a chemical substance or chemical composition means that the chemical substance or the chemical composition contains small amounts of impurities. In particular, the chemical substance or the chemical composition can contain impurities in a range of from 5 to 20 wt.%, preferably from 5 to 15 wt.%, more preferably from 5 to 10 wt.%, based on the total amount of the chemical substance or chemical composition. Particularly preferably, the chemical substance or the chemical composition contains from 1.5 to 5 wt.% of impurities. Impurities are understood as meaning all contents which differ from the chemical substance or the chemical composition. For example, technical grade ethanol can contain from 5 to 8 wt.% of impurities. This example cannot be generalized for all alcohols, rather the content of impurities with respect to the classification as "technical grade" is substance- or composition-related, or also depends on the preparation process. This classification according to the substance and the preparation process is familiar to the person skilled in the art.

It is likewise conceivable that it is not an individual alcohol or an individual technical grade alcohol which is employed as the alcohol component, but a mixture of several alcohols in the context of the abovementioned chemical composition. For example, several forms of the alcohol in accordance with that stated above can be employed as a mixture. Preferably, several alcohols characterized by at least one of the following features, such as different number of carbon atoms, different number of hydroxyl groups or different structure, or alcohols which differ simultaneously in two or more of the abovementioned features, such as can be obtained, for example, as technical grade products from large-scale industrial processes, are employed.

According to a preferred embodiment, monofunctional, difunctional, trifunctional, tetrafunctional or pentafunctional alcohols, or a mixture of two or more of these, are suitable as the alcohol component.

Alcohol components which are suitable in this connection are based, for example, on the following monofunctional alcohols: 1-propanol, 2-propanol, 1-butanol, 2-butanol, 2,2-dimethyl-1-propanol, 2-methyl-1-propanol, 2,2-dimethyl-1-propanol, 2-methyl-2-propanol, 2-methyl-1-butanol, 3-methyl-1-butanol, 2-methyl-2-butanol, 3-methyl-2-butanol, 1-pentanol, 2-pentanol, 3-pentanol, cyclopentanol, cyclopentenol, glycidol, tetrahydrofuryl alcohol, tetrahydro-2H-pyran-4-ol, 2-methyl-3-buten-2-ol, 3-methyl-2-buten-2-ol, 3-methyl-3-buten-2-ol, 1-cyclopropyl-ethanol, 1-penten-3-ol, 3-penten-2-ol, 4-penten-1-ol, 4-penten-2-ol, 3-pentyn-1-ol, 4-pentyn-1-ol, propargyl alcohol, allyl alcohol, hydroxyacetone, 2-methyl-3-butyn-2-ol, 1-hexanol, 2-hexanol, 3-hexanol, i-hexanol, 4-methyl-2-pentanol, 4-methyl-3-pentanol, 3-methyl-1-pentanol, 3-methyl-2-pentanol, cyclohexanol, 1-heptanol, 2-heptanol, 3-heptanol, 4-heptanol, i-heptanol, 5-methyl-2-hexanol, 5-methyl-3-hexanol, 5-methyl-4-hexanol, 4-methyl-1-hexanol, 4-methyl-2-hexanol, 4-methyl-3-hexanol, 3-methyl-1-hexanol, 3-methyl-2-hexanol, 1-octanol, 2-octanol, 3-octanol, 4-octanol, i-octanol, 2-ethylhexanol, 1-nonanol, 2-nonanol, 3-nonanol, 4-nonanol, 5-nonanol, i-nonanol, 1-decanol, 2-decanol, 3-decanol, 4-decanol, 5-decanol, 1-undecanol, 2-undecanol, i-undecanol, 1-dodecanol, 2-dodecanol, i-dodecanol, 1-tridecanol, 2-tridecanol, i-tridecanol, tetradecanol, hexadecanol, Guerbet alcohol, heptadecanol, 1-octadecanol, oleyl alcohol, eicosanol, behenyl alcohol, or two or more of these.

The following are suitable as the alcohol component based on difunctional alcohols: 1,2-ethanediol, 1,2-propanediol, 1,3-propanediol, dihydroxyacetone, 2-methyl-1,3-propanediol, 2-butyne-1,4-diol, 3-butene-1,2-diol, 2,3-butanediol, 1,4-butanediol, 1,3-butanediol, 1,2-butanediol, 2-butene-1,4-diol, 1,2-cyclopentanediol, 3-methyl-1,3-butanediol, 2,2-dimethyl-1,3-propanediol, 4-cyclopentene-1,3-diol, 1,2-cyclopentanediol, 2,2-dimethyl-1,3-propanediol, 1,2-pentanediol, 2,4-pentanediol, 1,5-pentanediol, 4-cyclopentene-1,3-diol, 2-methylene-1,3-propanediol, 2,3-dihydroxy-1,4-dioxane, 1,6-hexanediol, 2,5-hexanediol, 3,4-hexanediol, 1,2-hexanediol, 1,2-cyclohexanediol, 1,3-cyclohexanediol, 1,2-heptanediol, 1,7-heptanediol, 2,6-heptanediol, 3,4-heptanediol, 1,2-cycloheptanediol, 1,3-cycloheptanediol, 1,4-cycloheptanediol, 1,2-octanediol, 1,8-octanediol, 2,7-octanediol, 4,5-octanediol, 1,2-cyclooctanediol, 1,3-cyclooctanediol, 1,4-cyclooctanediol, 1,5-cyclooctanediol, 1,2-nonanediol, 1,9-nonanediol, 2-methyl-1,9-octanediol, 2,2-dimethyl-1,9-octanediol, or two or more of these.

The following are suitable as the alcohol component based on trifunctional alcohols: glycerol, 1,2,4-butanetriol, erythrose, threose, trimethylolethane, trimethylolpropane, 2-hydroxymethyl-1,3-propanediol or two or more of these.

The following are suitable as the alcohol component based on tetrafunctional alcohols: erythritol, threitol, pentaerythritol, arabinose, ribose, xylose, ribulose, xylulose, lyxose, ascorbic acid, gluconic acid γ-lactone, or two or more of these.

The following are suitable as the alcohol component based on pentafunctional and more highly functional alcohols: arabitol, adonitol, xylitol and dipentaerythritol.

According to a further preferred embodiment, the alcohol component is chosen from glycerol, glycerol dimer, glycerol trimer, glycerol tetramer, oligoglycerols, pentaerythritol, pentaerythritol dimer, pentaerythritol trimer, trimethylolpropane, bistrimethylolpropane, pentaerythritol, pentaerythritol dimer, n-butanol, i-butanol, n-propanol, i-propanol, 2,2-dimethylpropanol, 2-ethylhexanol, n-octanol, i-tridecanol, cetyl alcohol, stearyl alcohol, ethylene glycol, diethylene glycol, butyl glycol, dibutyl glycol, tributyl glycol, 2-propylheptanol, polyethylene glycol or two or more of these.

In this connection, reaction products of these alcohol components with ethylene oxide and/or propylene oxide are furthermore suitable, in each case independently between 2 and 30 units particularly preferably 2, 3, 4, 5, 6, 7, 8, 9 or 10 units of ethylene oxide and/or propylene oxide having been reacted on at least one, preferably two or more, particularly preferably all of the hydroxyl groups of the alcohol component. The use of di-, tri- or tetrabutyl glycol is moreover conceivable.

According to a further preferred embodiment, the alcohol component contains less than 10 wt.%, preferably less than 5 wt.% of nitrogen-containing compounds, based on the total weight of the alcohol component, nitrogen-containing compounds being both nitrogen-containing alcohol components and other nitrogen-containing organic compounds. The alcohol component furthermore preferably does not contain nitrogen atoms (N).

According to a further preferred embodiment, the alcohol component contains less than 10 wt.%, preferably less than 5 wt.% of aromatic ring compounds, based on the total weight of the alcohol component, aromatic ring compounds being both alcohols containing aromatic rings and other aromatic ring compounds. The alcohol component furthermore preferably does not contain aromatic ring compounds.

According to a further preferred embodiment, the alcohol component contains as non-metal atoms only non-metal atoms chosen from the group consisting of carbon (C), oxygen (O), nitrogen (N) or hydrogen (H) or several of these, preferably consisting of carbon (C), oxygen (O) and hydrogen (H).

In principle all carboxylic acids known to the person skilled in the art can be employed as the carboxylic acid component for the preparation of the ester. The term "carboxylic acid component" as used herein includes the carboxylic acid in its protonated form, the carboxylic acid in its deprotonated form, and also salts of the carboxylic acid, and also mixtures of at least two of the above, or of the carboxylic acid in its protonated form, its deprotonated form and at least one or more salts of the carboxylic acid.

The term "carboxylic acid component" furthermore in principle includes all compounds which contain at least one carboxylic acid group. This term also includes compounds which, in addition to the at least one carboxylic acid group, contain other functional groups, such as ether groups.

Carboxylic acid esters are preferably employed as the carboxylic acid component, the process for the preparation of an ester in this case being a transesterification. Suitable carboxylic acid esters are, in particular, those which are based on plant or animal oils or fats, e.g. tallow, such as, for example, beef tallow, kidney tallow or bovine kidney fat, lard, fish oil, neat's foot oil, seed oil, such as, for example, argan oil, apricot kernel oil (marillen kernel oil), cottonseed oil, borage oil (borage seed oil), thistle oil, groundnut oil, hazelnut oil, hemp oil, rose-hip kernel oil, elder seed oil, jojoba oil, currant seed oil, coconut oil/coconut fat, kukui oil, kiwi seed oil, pumpkin seed oil, linseed soil, cameline oil, macadamia oil, almond oil, poppy oil, evening primrose oil, palm oil, palm kernel oil, peach kernel oil, rape oil, rice oil, castor oil, sea buckthorn kernel oil, mustard oil, nutmeg flower oil, sesame oil, shea oil, shea butter, soya oil, sunflower oil, walnut oil, grape seed oil, wheat germ oil or cedar oil, fruit pulp fats, such as, for example, olive oil, palm oil, avocado oil or sea buckthorn oil, or also germ oils, such as, for example, rape germ oil, wheat germ oil, maize germ oil, rice germ oil, rice husk oil, soya germ oil or sunflower germ oil, or a mixture of two or more of these. The use of tallow, of rape oil and of coconut, canola, palm, soya or sunflower oil is most preferred.

A technical grade carboxylic acid can furthermore also be employed as the carboxylic acid component. It is accordingly preferable for not an individual carboxylic acid or an individual technical grade carboxylic acid to be employed as the carboxylic acid component, but a mixture of several carboxylic acids. For example, several forms of the carboxylic acid in accordance with that stated above can be employed as a mixture. Preferably, several carboxylic acids characterized by at least one of the following features, a different number of carbon atoms, a different number of carboxylic groups or a different structure, or carboxylic acids which differ simultaneously in several of the abovementioned features, such as can be obtained, for example, as technical grade products from large-scale industrial processes, are employed. The substance-related amount of impurities in the technical grade is familiar to the person skilled in the art.

The use of mono-, di- or polycarboxylic acids is preferred according to the invention.

Possible carboxylic acid components are, in particular, saturated or unsaturated carboxylic acids, acid chlorides of the carboxylic acids and acid anhydrides of the carboxylic acids having a number of carbon atoms in a range of from 6 to 26, particularly preferably in a range of from 8 to 24, still more preferably in a range of from 10 to 22, moreover preferably in a range of from 12 to 20 and most preferably in a range of from 14 to 18. The carboxylic acid components furthermore preferably have from 8 to 12 C atoms.

Carboxylic acid components which are suitable in this connection are, for example, derived from the following monocarboxylic acids: acetic acid, propionic acid, butyric acid, valeric acid, caproic acid, oenanthic acid, caprylic acid (octanoic acid), i-octanoic acid, pelargonic acid (nonanoic acid), capric acid (decanoic acid), lauric acid (dodecanoic acid), myristic acid, palmitic acid, margaric acid, stearic acid, arachic acid, behenic acid or also unsaturated carboxylic acids, such as e.g. acrylic acid, methacrylic acid, 3-butenoic acid, 4-pentenoic acid, 5-hexenoic acid, 6-heptenoic acid, 7-octenoic acid, 8-nonenoic acid, 9-decenoic acid, undecylenic acid, palmitoleic acid, oleic acid, elaidic acid, vaccenic acid, icosenic acid, ricinoleic acid, 12-hydroxystearic acid, cetoleic acid, erucic acid, and polyunsaturated carboxylic acids, for example linoleic acid, linolenic acid, arachidonic acid, timnodonic acid, clupanodonic acid or cervonic acid.

Suitable carboxylic acid components based on dicarboxylic acids are, for example, malonic acid, maleic acid, fumaric acid, succinic acid, glutaric acid, adipic acid, pimelic acid, suberic acid, azelaic acid, sebacic acid, tartaric acid, malic acid, α-ketoglutaric acid, oxaloacetic acid, o-phthalic acid, m-phthalic acid or p-phthalic acid. Examples of a suitable tricarboxylic acid are trimellitic acid or citric acid. The use of a mixture of at least two of the abovementioned carboxylic acid components is furthermore conceivable.

According to a preferred embodiment, the carboxylic acid component is chosen from octanoic acid, i-octanoic acid, caprylic acid, nonanoic acid, i-nonanoic acid, 9-decenoic acid, decanoic acid, i-decanoic acid, sebacic acid, palmitic acid, stearic acid, oleic acid, azelaic acid, pelargonic acid, trimellitic acid, adipic acid, erucic acid, behenic acid, HOOC-C₃₆H₇₂-COOH, phthalic anhydride, or a mixture of two or more of these..

According to a further preferred embodiment, the carboxylic acid component contains exactly one carboxyl group.

According to a further preferred embodiment, the carboxylic acid component contains less than 10 wt.%, preferably less than 5 wt.% of nitrogen-containing compounds, based on the total weight of the carboxylic acid component, nitrogen-containing compounds being both nitrogen-containing carboxylic acids and other nitrogen-containing organic compounds. The carboxylic acid component furthermore preferably does not contain nitrogen atoms (N).

According to a further preferred embodiment, the carboxylic acid component contains less than 10 wt.%, preferably less than 5 wt.% of aromatic ring compounds, based on the total weight of the carboxylic acid component, aromatic ring compounds being both carboxylic acids containing aromatic rings and other aromatic ring compounds. The carboxylic acid component furthermore preferably does not contain aromatic ring compounds.

According to a further preferred embodiment, the carboxylic acid component contains as non-metal atoms only non-metal atoms chosen from the group of carbon (C), oxygen (O), nitrogen (N) or hydrogen (H), carbon (C), oxygen (O) or hydrogen (H), or several of these.

According to a further preferred embodiment, the carboxylic acid component contains less than 10 wt.%, preferably less than 5 wt.% of compounds containing hydroxyl groups, based on the total weight of the carboxylic acid component, compounds containing hydroxyl groups being both hydroxycarboxylic acids, and other organic compounds containing hydroxyl groups. The carboxylic acid component furthermore preferably does not contain hydroxyl groups.

According to a further preferred embodiment, the carboxylic acid component comprises a mixture of adipic acid, or an adipic acid derivative, as a dicarboxylic acid, and at least one monocarboxylic acid.

"Pure" and "technical grade oleic acid" can be employed as oleic acid. A pure oleic acid is understood as meaning a composition which contains more than 98 wt.% of oleic acid. A "technical grade oleic acid" is understood as meaning a composition which contains oleic acid to the extent of 98 wt.% or less. Such a technical grade oleic acid contains e.g. oleic acid in a range of from 60 to 75 wt.%, linoleic acid in a range of from 5 to 20 wt.% and stearic acid in a range of from 0 to 5 wt.%, based on the total weight of the technical grade oleic acid, the sum of the percentages by weight being 100. A suitable technical grade oleic acid is marketed e.g. by Cognis Oleochemicals GmbH, Germany, under the name "Edenor TiO5". Such a technical grade oleic acid which can preferably be employed can be obtained from animal fats, for example beef tallow. A technical grade oleic acid with a higher content of oleic acid can likewise be employed, e.g. with 80 to 95 wt.%, preferably 85 to 95 wt.% and furthermore preferably 90 to 95 wt.%, in each case based on the total composition. A technical grade oleic acid with 96 to 98 wt.% of oleic acid, based on the total composition, is very particularly preferred. Another technical grade oleic acid with approx. 80 to 90 wt.% of oleic acid, 2 to 10 wt.% of linoleic acid, 2 to 6 wt.% of stearic acid and 2 to 6 wt.% of palmitic acid, based on the total weight of the other technical grade oleic acid, is furthermore preferred, the sum of the percentages by weight being 100. Such another technical grade oleic acid is marketed e.g. as "high oleic" sunflower oil or HO sunflower oil.

According to a further preferred embodiment, a palm oil complex ester comprising a mixture of adipic acid, palmitic acid and stearic acid as the carboxylic acid component and pentaerythritol as the alcohol component can be prepared. Preferably, a mixture of 10 to 30 wt.% of adipic acid, 30 to 45 wt.% of palmitic acid and 40 to 50 wt.% of stearic acid, based on the carboxylic acid component, can be employed, the sum of the percentages by weight being 100. It emerges from this that a complex ester in the context of this invention is a mixture of two or more individual esters which, although generally present in the pure form, can occasionally contain small amounts of impurities which differ from esters.

According to a further preferred embodiment, an ester II comprising 10 to 40 wt.% of phthalic anhydride as the carboxylic acid component and 60 to 90 wt.% of a C_{16/18}-fatty alcohol mixture as the alcohol component can be employed, the sum of the percentages by weight being 100.

According to a further preferred embodiment, an ester III comprising 25 to 75 wt.% of a C_{16/18}-carboxylic acid fraction as the carboxylic acid component and 75 to 25 wt.% of a C_{16/18}-fatty alcohol mixture as the alcohol component can be employed, the sum of the percentages by weight being 100.

According to a further preferred embodiment, an ester IV comprising 60 to 99 wt.% of a C_{16/18}-carboxylic acid fraction as the carboxylic acid component and 1 to 40 wt.% of pentaerythritol as the alcohol component can be employed, the sum of the percentages by weight being 100.

According to a further preferred embodiment, an ester V comprising 60 to 90 wt.% of a C_{16/18}-carboxylic acid fraction as the carboxylic acid component and 1 to 30 wt.% of pentaerythritol and 1 to 30 wt.% of adipic acid as the alcohol component can be employed, the sum of the percentages by weight being 100.

The process according to the invention for the preparation of an ester from a carboxylic acid component and an alcohol component can be carried out in the presence of further additives, for example one or more catalysts, stabilizers, antioxidants, viscosity regulators and mixtures thereof.

The process according to the invention is preferably carried out in the presence of a catalyst. In principle, any compound which is known from the abovementioned catalyst groups to the person skilled in the art and appears to be suitable for catalysis of the esterifications according to the invention is suitable here as the catalyst. Preferably, several catalysts of the abovementioned catalyst groups are employed as a catalyst mixture.

Preferably, the catalyst or a catalyst mixture is employed in a range of from 0.0001 to 5 wt.%, preferably from 0.0005 to 4 wt.%, further preferably from 0.001 to 3.5 wt.%, moreover preferably from 0.004 to 3.0 wt.%, in each case based on the total amount of process components a. and b. Particularly preferably, the amount of catalyst added is in a range of from 0.006 to 2.5 wt.%, from 0.008 to 2.5 wt.%, from 0.01 to 2.0 wt.%, from 0.03 to 1.8 wt.%, from 0.05 to 1.6 wt.% or from 0.08 to 1.3 wt.%, in each case based on the total amount of process components a. and b. A range of from 0.1 to 1.2 wt.%, from 0.2 to 1.1 wt.%, from 0.3 to 1.0 wt.% or from 0.4 to 0.9 wt.%, in each case based on the total amount of process components a. and b., is still more preferable.

If the catalyst is a solid at room temperature, the catalyst is preferably present in the form of particles, for example in the ground form. A particle size in a range of from 30 µm to 2 mm is preferred here. In accordance with that stated above, preferably at least 40 wt.%, in particular at least 45 wt.%, particularly preferably at least 50 wt.%, and most preferably a range of from at least 40 wt.% to 60 wt.% of the particles, in each case based on the total weight of the catalyst, have a particle size in the ranges described above.

According to a further preferred embodiment, at least 70 wt.%, particularly preferably at least 80 wt.%, or at least 90 wt.%, up to 95 wt.%, or 98 wt.% of the catalyst, based on the total amount of catalyst, is present in the reactor. In accordance with that stated above, in a particularly preferred embodiment the catalyst is not configured as a fixed bed catalyst, or is not bound in a polymer matrix, or is not absorbed in a zeolite, or is not applied to a support surface.

At least one compound chosen from the group consisting of proton donor or electron donor, or both, can advantageously be employed as the catalyst.

Suitable catalysts from the group of proton donors are, for example, sulphuric acid or phosphoric acid, aliphatic or aromatic sulphonic acids, such as methanesulphonic acids or benzenesulphonic acids, such as o- or m-toluenesulphonic acid, particularly preferably p-toluenesulphonic acid or methanesulphonic acid. It is likewise conceivable to employ fluorinated aliphatic or aromatic sulphonic acids, particularly preferably trifluoromethanesulphonic acid.

Suitable catalysts from the group of electron donors are, preferably, metals, metal compounds or reducing acids. Suitable metals are, in particular, tin, titanium, zirconium, which are preferably employed as finely divided metal powders. Suitable metal compounds are the salts, oxides or soluble organic compounds of the metals described above, or a mixture of at least two of these. In contrast to the proton donors, the metal compounds are high temperature catalysts which as a rule achieve their full activity only at temperatures above 180 °C. They are preferred according to the invention because a smaller amount of by-products, such as, for example, olefins, are formed compared with catalysis with proton donors. Catalysts which are particularly preferred according to the invention are a) one or more divalent tin compounds, or b) one or more tin compounds and elemental tin, which can react with the educts to give divalent tin compounds. For example, tin, tin(II) chloride, tin(II) sulphate, tin(II) alcoholates or tin(II) salts of organic acids, in particular of mono- and dicarboxylic acids, e.g. dibutyltin dilaurate, dibutyltin diacetate, or a mixture of at least two of these, can be employed as the catalyst. Particularly preferred tin catalysts are tin(II) oxalate and tin(II) octoate.

In principle all reducing acids which are known to the person skilled in the art and appear to be suitable are suitable as catalysts of the group of reducing acids.

Hypophosphorous acid, sulphurous acid, phosphorous acid, selenious acid, oxalic acid, ascorbic acid, or two or more of these are particularly preferred.

According to a further preferred embodiment, a mixture which comprises at least two, in particular at least three catalysts from one or more of the abovementioned groups is employed as the catalyst. Particularly preferably, two or more catalysts are chosen, each catalyst being chosen from in each case different abovementioned groups.

According to a further preferred embodiment, a catalyst mixture comprising at least two different catalysts is employed, the first catalyst being chosen from the group of proton donors and the at least one further catalyst being chosen from the group of electron donors, or a mixture of two or more of these. Such a catalyst mixture can have a high catalytic activity at temperatures which are lower compared with the high temperature catalysts, e.g. between 140 and 180 °C, or between 120 and 185 °C. At the same time, because of the lower process temperature, a smaller amount of by-products which appear coloured, in particular a smaller amount of substances which cause a yellowish or brownish colouring, is formed. A mixture comprising p-toluenesulphonic acid and a tin compound is particularly preferred as the catalyst mixture. According to a further preferred embodiment, the catalyst or the catalyst mixture does not contain tin oxide.

According to a further preferred embodiment, a mixture of 0.001 to 1 wt.% of an electron donor of the group of metal or metal compound, 0.001 to 1 wt.% of a proton donor and 0.001 to 1 wt.% of a second electron donor from the group of reducing acid, in each case based on the total amount of process components a. and b., can be employed as the catalyst. Particularly preferably, tin oxalate is chosen as the metal compound, p-toluenesulphonic acid is chosen as the proton donor and hypophosphorous acid is chosen as the reducing acid.

According to a further preferred embodiment, a catalyst which comprises one or more compounds chosen from the group consisting of sodium hydroxide, potassium hydroxide, lithium hydroxide, magnesium hydroxide, calcium hydroxide and strontium hydroxide is employed as an additive. Such a catalyst is particularly preferred if a carboxylic acid ester is particularly preferably chosen as the carboxylic acid component.

According to a further preferred embodiment of the process according to the invention, the at least one ester has between 1 and 6 ester groups.

In the context of carrying out the process according to the invention, process components a., b., d. and optionally c. are first employed in process step i. The sequence and the nature and manner of the addition of the individual components a., b., d. and optionally c. into the reactor in principle is not critical. Preferably, all the process components required for a reaction which are to be attributed to one of the groups chosen from alcohol component, carboxylic acid component and catalyst are in each case introduced into the reactor as process components within the particular group at least partly at the same time. In this context, the carboxylic acid components and alcohol components envisaged for the preparation of the ester according to the invention can be initially introduced and can then be reacted in the presence of a suitable catalyst or a suitable catalyst mixture. Furthermore, in a preferred embodiment the catalyst components are initially introduced together with one of the process components chosen from one of the groups of alcohol components or carboxylic acid components and the other components are then added. If the catalyst components are introduced into the reactor together with a process component, this can be effected by simultaneous introduction, and by introduction as a mixture, solution, suspension or dispersion.

Process components a., b., d. and the additives c. are provided in the reactor in liquid or in solid form. It may be preferable in the case of process components to be provided which are solid at the ambient temperature to be liquefied by heating. It is conceivable both that the liquefying is carried out in the course of providing the components, e.g. by means of a preheating stage, and that these process components are stored in liquid form at elevated temperature and are led from the holding place under thermostatic control and in an insulated line through a metering device. The addition of the process components in liquid form makes simple metering possible and promotes swift mixing of the process components introduced into the reactor.

Suitable metering devices are in principle all the devices which are known to the person skilled in the art and appear to be suitable. Electrically controllable shut-off valves or delivery pumps are particularly suitable.

The addition of the additives c. is in general carried out in a separate step to the components a., b. and d. already initially introduced. If these are solids, these are preferably introduced through a sluice at the upper side of the reactor, the contents of the reactor being stirred vigorously. A cellular wheel sluice can particularly preferably be employed as the sluice. It is often advantageous to mix the components, while stirring, in the context of providing them.

If at least one catalyst or a catalyst mixture is employed as an additive, a mixture of solids, a suspension or a liquid mixture is preferably employed. Preferably, the catalyst or the catalyst mixture is added only at the start of the reaction.

The reaction of the process components in process step ii. of the process according to the invention can be carried out by all processes which are known to the person skilled in the art and appear to be suitable. In this context, it may be advantageous to remove the water formed in the reaction from the reaction mixture, this removal of the water preferably being carried out by distillation, optionally by distillation with alcohol employed in excess, during the reaction.

Alcohol which has not reacted after the reaction has been carried out can also be removed from the reaction mixture, this removal of the alcohol preferably being carried out by means of distillation. When the reaction has ended, in particular after the unreacted alcohol has been separated off, the catalyst present in the reaction mixture can furthermore be separated off by washing with water, a filtration or by centrifugation, optionally after treatment with a base.

It is furthermore preferable to carry out the reaction at a temperature in a range of from 50 to 300 °C, particularly preferably in a range of from 100 to 250 °C and very particularly preferably in a range of from 100 to 280 °C, most preferably in a range of from 170 to 250 °C and furthermore preferably in a range of from 140 to 200 °C. The preferred temperatures depend on the alcohol component chosen, the progress of the reaction, the catalyst type and the catalyst concentration. These can be easily determined by experiments for each individual case. Higher temperatures increase the rates of reaction and promote side reactions, for example splitting off of water from alcohols or the formation of coloured by-products or both.

It is furthermore preferable to carry out the reaction of the process components at a temperature in a range of from 50 to 160 °C, particularly preferably in a range of from 80 to 150 °C and very particularly preferably in a range of from 100 to 140 °C, most preferably in a range of from 120 to 140 °C. Preferably, proton acids are then employed as the catalyst or catalyst mixture. Particularly preferably, no further catalysts are then added.

It is furthermore preferable to keep the process components uniformly mixed by stirring during the reaction.

It is furthermore preferable in connection with the process according to the invention for the ester A obtained in the reaction ii. to be after-treated.

"After-treatment" is understood as meaning all conceivable steps and processes which are familiar to the person skilled in the art and which can be undertaken in order to purify the ester A obtained in process step ii. from by-products, impurities, catalysts and other additives or those processes with which the ester A is further processed to an end product.

These are understood as meaning, in particular, distillation, sorption, filtration, bleaching, centrifugation, washing, crystallization or drying processes, and continuing reactions, or a combination of at least two or more of these. Pressure filtration, bleaching and spray drying processes are preferred.

In connection with the esters which can be prepared from a carboxylic acid component and an alcohol component with several hydroxyl groups in the process according to the invention, it is furthermore preferable for not all the hydroxyl groups of the alcohol component to be esterified, so that some of the hydroxyl groups remain non-esterified. In this connection, it is particularly preferable for from 5 to 80 mol%, particularly preferably from 10 to 70 mol%, still more preferably from at least 20 to 50 mol%, moreover preferably from 30 to 40 mol% and most preferably from 45 to 55 mol% of the hydroxyl groups of the alcohol component not to be esterified. This means that in the ester obtainable by reaction of the composition according to the invention, the content, described in mol%, of all the hydroxyl groups originally present in the alcohol component containing several hydroxyl groups for the preparation of the ester from a carboxylic acid component and an alcohol component is not esterified, and thus is also present as hydroxyl groups in ester A, and optionally also in ester B.

According to the invention, the ester A is modified into a particulate ester in granule form during the after-treatment, the after-treatment comprising a process for the preparation of an ester in granule form in a spray or trickle tower, with at least the following after-treatment steps:
aa. provision of the ester as a fluid stream,
bb. optionally addition of auxiliary substances,
cc. charging of the fluid stream with pressure,
dd. release and division of the fluid stream via a pressure release device into a contact region to give a discontinuous fluid stream and
ee. cooling of the discontinuous fluid stream in the contact region by a cooling fluid in counter-current, to give a solid particle stream,
wherein according to formula (I) a ratio *I₁* of the pressure *p_{E}* of the fluid stream before the pressure release device to the difference between the exit temperature *T_{2,CF}* out of and entry temperature *T_{1,CF}* of the cooling fluid into the contact region is chosen in a range of from 0.05 to 4.0, further preferably from 0.1 to 3.5, or from 0.3 to 3, or from 0.5 to 2.5, or from 0.6 to 1.5 or from 1.0 to 1.5, ${I}_{1} = \left|\frac{{p}_{E}}{{T}_{2 , CF} - {T}_{1 , CF}}\right|$
or
wherein according to formula (II) a ratio *I₂* of the difference from the entry temperature *T_{1,E}* of the ester A present as a discontinuous fluid stream into the contact region to the melting point *T_{m,E}* of the ester A and the difference between the exit temperature *T_{2,CF}* out of and entry temperature *T_{1,CF}* of the cooling fluid into the contact region is chosen in a range of from 0.04 to 3.0, further preferably from 0.05 to 2.3, or from 0.08 to 2.0, or from 0.1 to 1.5, or from 0.1 to 1.2, ${I}_{2} = \left|\frac{{T}_{1 , E} - {T}_{m , E}}{{T}_{2 , CF} - {T}_{1 , CF}}\right|$
or
wherein a ratio *I₃* (III) of the pressure *p_{E}* of the fluid stream before the pressure release device to the viscosity η*_{E}* of the fluid stream before the pressure release device is chosen in a range optionally of from 0.04 to 0.95, further preferably from 0.08 to 0.85, or from 0.1 to 0.75, or 0.2 to 0.6, ${I}_{3} = \frac{{p}_{E}}{{η}_{E}}$
where
p_{E} = pressure of the fluid stream before the pressure release device, determined in [bar];
η_{E} = viscosity of the fluid stream before the pressure release device, determined in [mPa·s],
or
a combination of two or more of these. The unit of *I₃* is [mPa·s / bar].

Preferably, the ester granulation is the last stage, if the after-treatment envisages several after-treatment stages.

The provision of the ester A as a fluid stream according to process step aa. is preferably effected by heating the ester A to an after-treatment temperature above the melting temperature of the ester A, preferably at least 5 K, or at least 10 K above this. If the ester A is already present as a fluid, for example from a preceding process step, where appropriate it is heated further to the desired after-treatment temperature or, if the temperature of the fluid is higher than that of the after-treatment, cooled. Auxiliary substances can optionally be added according to process step bb.

According to process step cc., the fluid stream obtained in this way is charged with a pressure, preferably with a pressure in a range of from 1,500 to 40,000 mbar, if atomization is intended, particularly preferably from 3,500 to 25,000 mbar, or from 5,000 to 10,000 mbar. In a trickle process, the pressure is preferably in a range of from 1,500 to less than 3,500 mbar, particularly preferably from 1,5000 to 3,000 mbar, or from 1,500 to 2,800 mbar. This can be effected by any method and manner which is known to the person skilled in the art and appears to be suitable, for example by applying a gas pressure, generating pressure by compression of the fluid by means of a pump or a compressor station, reducing the cross-section of the fluid stream, gravity pressure of a static column, or a combination of two or more of these. Charging with pressure by compression of the fluid with a pump or a compressor station is preferred in particular.

The fluid stream charged with pressure is then released via a pressure release device into a contact region to give a discontinuous fluid stream in step dd. Before the fluid stream passes through the pressure release device, the viscosity of the fluid stream η_{E}, which has the temperature T_{1,E}, is preferably in a range of from 2 to 1,000 mPa·s, preferably 5 to 500 mPa·s, or from 10 to 240 mPa·s, or from 5 to 150 mPa·s, or from 10 to 100 mPa·s. This viscosity is furthermore preferably in a range of from 10 to 60 mPa·s, or 5 to 40 mPa·s.

Generally, the contact region is demarcated at the top by the opening of the pressure release device and at the bottom by the inlet or the inlets of the cooling fluid. In principle any pressure release device which is known to the person skilled in the art and appears to be suitable is suitable as the pressure release device. A nozzle is particularly preferred here. In principle any nozzle which is known to the person skilled in the art and appears to be suitable for this use is suitable as the nozzle. Full cone nozzles with at least one of the following features are particularly preferred:
- M1:: a rotary insert
- M2:: an apex angle of from 30° to 60°, particularly preferably 40° to 50°, or
- M3:: a pitch of the nozzle with respect to the perpendicular in a range of from -30 to +30°, preferably -20 to +20°,
- M4:: an opening with a minimum diameter in a range of from 1 to 8 mm, preferably from 2 to 5 mm, or from 3 to 4 mm.

Furthermore, in particularly preferred embodiments, two or more of the above features are combined. The following embodiments which are represented with the aid of the combinations of figures thus result specifically: M1M2, M1M3, M1M4, M2M3, M2M4, M3M4, M1M2M3, M1M2M4, M1M3M4, M2M3M4, and M1M2M3M4.

According to a further preferred embodiment, several nozzles connected by fluid-conducting means are employed in a parallel arrangement. For example, 2, 3, 4, 5, 6, 7, 8, 9, 10, 11 or 12 nozzles can be employed side by side. The throughput during the after-treatment can be increased accordingly in this manner.

According to a further preferred embodiment, the throughput of the fluid stream at any desired point in the process, particularly preferably in process step cc., dd., or both, is in a range of from 100 to 9,000 kg/h, particularly preferably 500 to 8,000 kg/h, or from 1,000 to 5,000 kg/h, or from 2,000 to 4,500 kg/h, or from 2,500 to 4,000 kg/h.

If, for example, a nozzles is suitable for a throughput of from 100 to 1,000 kg/h, in accordance with that described above several nozzles can be employed in a parallel arrangement in order to achieve a total throughput during the after-treatment of, for example, 3,000 or 4,000 kg/h.

According to a further preferred embodiment, the flow rate of the cooling fluid through the contact region is in a range of from 5,000 to 50,000 m³/h, particularly preferably from 10,000 to 30,000 m³/h, further preferably from 15,000 to 30,000 m³/h.

The discontinuous fluid stream obtainable in step dd. is next cooled in step ee. in the contact region by a cooling fluid in counter-current, to give a solid particle stream. It may be sufficient here for a counter-current to be present only in a part of the contact region.

According to a further preferred embodiment, the distance in the contact region between the entry and exit of the fluid stream is in a range *h* of from 10 to 50 m, particularly preferably 15 to 30 m.

According to a further preferred embodiment, step ee. is carried out under a reduced pressure in the contact region of from 2 to 900 mbar, preferably from 100 to 600 mbar.

According to a further preferred embodiment, the temperature of the cooling fluid on entry into the contact region T_{1,CF} is in a range of from -5 to +30 °C, or from -5 to +5 °C.

According to a further preferred embodiment, the temperature of the cooling fluid on exit from the contact region T_{2,CF} is in a range of from +15 to +50 °C, or from +20 to +40 °C.

According to a further preferred embodiment, the ratio according to formula (IV) of the pressure of the fluid stream before the release at the pressure release device to the pressure of the fluid stream after release in the contact region is in a range of from 1.5:1 to 50:1 (bar/bar), or from 3:1 to 40:1, or from 4:1 to 30:1 or from 4:1 to 15:1, or from 4.5:1 to 9:1. ${I}_{4} = \frac{{p}_{E}}{{p}_{C}}$
where
p_{E} = pressure of the fluid stream before the release;
p_{C} = pressure in the contact region.

For the purposes of the present invention, the particle size is determined as the dimension of a particle of the particle stream, this dimension being determined by sieve analysis. Thus, for example, particles which are retained by a sieve with openings of a width of 710 µm are particles with a particle size of greater than 710 µm. Particles which are allowed through by this sieve but are retained by a sieve with openings of a width of 500 µm are consequently particles with a particles size of greater than 500 µm.

The mass mean particle size of a sample of particles or of a plurality of particles is furthermore determined as that particle size at which the amount of particles is divided into two halves in terms of weight. This means that the one half of the particles in terms of weight have particle sizes of more than the mass mean particle size, and the other half in terms of weight have particle sized of less than the mass mean particle size. The mass mean particle size of a sample of particles or of a plurality of particles is thus 500 µm if the one half of the particles in terms of weight are retained by a sieve with openings of 500 µm width, and the other half of the particles are allowed through by this sieve. A typical method for determination of the particle size in the form of a graph (in which the percentages by weight of particles of a sample of particles or of a plurality of particles retained or allowed through by a sieve of given opening width are plotted cumulatively against the width of the sieve opening of several sieves on probability paper) is typically used to determine the mass mean particle size if the 50 per cent mass value does not coincide with the width of a USA standard test sieve. A plot of this type is also typically used to determine the distribution of the particle size at a certain mass mean.

According to a further preferred embodiment of the process according to the invention, the particles obtainable as a particle stream in step ee., taking into account the above statements, have a particle size distribution which is determined as follows:
- a mass mean particle size in the range of from 400 to 800 µm, preferably from 500 to 700 µm; and
- up to 20 wt.%, or particularly preferably up to 15 wt.%, of these particles with a particle size of less than 200 µm; and
- up to 20 wt.%, or particularly preferably up to 15 wt.%, of these particles with a particle size of more than 1,000 µm.

The particle size is particularly preferably determined at a temperature of the particles of 20 °C.

In agreement with the above description of the mass mean particle size, further preferred and advantageous particle size characteristics of the particle stream particles can be determined by means of the standard sieve analysis described here. In such a typical sieve analysis, a sample or a plurality of particles of the particle stream is sieved through a number of sieves with decreasing size of the openings, the percentages by weight of the sample which are retained or allowed through being determined. Such test methods have been standardized, for example, by the American Society for Testing Materials (ASTM).

A Tyler Ro-Tap sieve machine (produced by W.S. Tyler, Inc.) and a sieve series according to the "US Sieve Series" or "Tyler Standard Sieve Series" is used in accordance with one of the abovementioned methods. The determination of the particle size distribution using this technique is described in detail in "Perry's Chemical Engineers' Handbook", 6th edition, (McGraw-Hill Book Company, 1984), pages 21-13 to 21-19.

The process for the preparation of the esters according to the present invention can be carried out in a reaction region comprising a reactor. In principle, all reactor types known to the person skilled in the art which this person considers suitable for carrying out the process according to the invention can be employed. Preferably, a stirred tank on the side wall of which is arranged a heating jacket on the outside or inside is employed as the reactor. The heating jacket can be arranged on a part of the side wall or on the entire side wall. Preferably, the heating jacket is arranged on the entire side wall. Furthermore, the heating jacket particularly preferably can be controlled in sections. For example, the heating jacket is in 3, 4, 5 or more sections, each of which can be heated independently of each other. For transportation of heat, a heat transfer medium is led to the heating jacket through heating lines. All the usual heat transfer media known to the person skilled in the art are suitable as the heat transfer medium. The heat transfer medium can be either a heating means or a coolant. The heat transfer medium can also be under pressure. Preferably, heating steam, thermal oil or water, particularly preferably heating steam, is chosen as the heat transfer medium.

Furthermore, the reactor advantageously has a stirrer with a stirrer motor, transmission and stirrer shaft with stirrer blades, which is arranged on the upper side of the stirred tank, preferably centrally. The length of the stirrer shaft, the number of stirrer tiers arranged on the stirrer shaft, the diameter of these stirrer tiers and the geometry of the stirrer blades arranged in each stirrer tier are advantageously chosen such that during operation a uniform mixing of the process components, and where appropriate of the reaction products, is ensured, especially in the regions close to the base. The length of the stirrer shaft is preferably chosen such that the stirrer shaft extends from a motor lying outside the reactor, or from a transmission driven by a motor, almost to the base of the reactor. Preferably, the length of the stirrer shaft is chosen such that a distance of between about 5 to about 10 %, with respect to the height of the reactor tank, remains between the end of the shaft and the reactor base. The stirrer shaft can be mounted on one side or, if the stirrer shaft is constructed to the reactor base, mounted at two points.

All stirrer types known to the person skilled in the art which this person considers suitable for carrying out the process according to the invention can be employed as stirrers. Preferably, stirrer types which have the effect at least in part of axial mixing during operation can be employed in particular. The stirrers can have one or more stirrer tiers, preferably one, 2, 3, 4, 5, 6 or 7 tiers. With respect to the geometry, cross, angled blade or disc stirrers with suitable stirrer blades are particularly preferred, and MIG or INTERMIG stirrers are most preferred. In the case of angled blade, disc and MIG stirrers, the stirrer blades in adjacent tiers can be displaced by 90° in the horizontal plane. The stirrers particularly preferably have an even number of tiers.

The stirrers are preferably produced from steel, preferably from V2A or V4A steel, particularly preferably from the following materials, the material numbers being found in EN10088: 1.4307, 1.4306, 1.4311, 1.4301, 1.4948, 1.4404, 1.4401, 1.4406, 1.4432, 1.4435, 1.4436, 1.4571, or 1.4429, particularly preferably 1.4301 or 1.4571.

The stirrer can moreover be at least partly coated with a surface coating composition. Preferably, the stirrer is equipped with a polymer coating. For example, a fluoropolymer coating which protects the material from which the stirrer is made from the fluid or mixture to be stirred is suitable as a polymer coating.

Preferably, a ratio of the diameter of the stirrer tier(s) to the diameter of the reactor of from 0.55 to 0.75, particularly preferably 0.60 to 0.70 or 0.62 to 0.68, very particularly preferably 0.64 to 0.66, e.g. 0.65, is chosen. By suitable choice of the parameters, the person skilled in the art ensures complete mixing and mingling in the reactor and avoids a deposit of solid constituents.

The stirrer blades can have the most diverse geometries, the geometry influencing the nature of the mixing. The "nature of the mixing" is understood as meaning the polar vector acting on the stirred mixture due to the movement of the stirrer. The polar vector has vertical and horizontal contents. Usually, both contents are not equal to zero. For example, a cross stirrer with stirrer blades arranged axially to the stirrer shaft and aligned vertical to the stirrer plane has the effect of rather horizontal mixing, whereas a cross stirrer with stirrer blades arranged at an angle, e.g. axially, to the stirrer shaft and at an angle of 30°, 45° or 60° with respect to the stirrer plane has the effect of a more vertical mixing. It is furthermore conceivable to provide a spiral stirrer.

Stirrers of which the stirrer blades have, with respect to the stirrer plane, a positive incline in the region close to the stirrer shafts, preferably the inner two thirds of the stirrer blade, and a negative incline in the region away from the stirrer shafts, preferably the outer third of the stirrer blade, are particularly preferred.

The incline of a stirrer blade is understood as meaning its alignment with respect to the stirrer plane, a positive incline meaning that the stirrer blade rises in the direction of rotation from its front edge from the bottom upwards to its rear edge, and has the effect of an ascending flow of material. A negative incline means that the stirrer blade drops in the direction of rotation from its front edge from the top downwards to its rear edge, i.e. has the effect of a descending flow of material. Such a stirrer has the effect of vertical mixing from the bottom upwards in the region of the middle of the reactor and a vertical mixture from the top downwards at the reactor wall.

The type of mixing described above can be assisted and adapted with further auxiliary devices. For example, baffles can be provided on the inside wall of the reactor. These are preferably attached to the inside wall of the reactor in the vertical direction, the plane in which the baffle lies being aligned through or at least in the direction of the vertical axis of the reactor.

According to another example, end stirrer organs which are moved a short distance above the reactor base can be attached on the lower stirrer tier. A short distance is to be understood as meaning so small that deposits of solid on the bottom can be carried along and/or swirled up by the stirrer. In this context, the end stirrer organs have the effect of a horizontal mixing to the extent of at least 50 %, preferably 70 %, based on the layer mixed by the end stirrer organs. The end stirrer organs preferably have a flat shape, the sides of the flat shape which are adjacent to the reactor base and the reactor wall being designed such that an essentially constant gap is provided between the reactor base and the reactor wall. If the reactor base is curved, for example, the end stirrer organs have a surface which is at least rounded at the side, and optionally an angled position of the end stirrer organs. Preferably, the end stirrer organs sweep over the reactor base at a distance of from 10 to 30 cm, preferably 15 to 25 cm or 30 cm.

In principle all materials which are known to the person skilled in the art and which this person considers suitable with respect to carrying out the process according to the invention, in particular with respect to strength, elasticity and corrosion resistance, can be employed as the material for production of the devices described above. In particular, the materials which are preferred in the choice of material for the stirrer are also preferred. Rustproof steel, preferably V2A or V4A steel, in particular the following materials, are preferably employed for production of the reactor, the material numbers being found in EN10088: 1.4307, 1.4306, 1.4311, 1.4301, 1.4948, 1.4404, 1.4401, 1.4406, 1.4432, 1.4435, 1.4436, 1.4571, or 1.4429, particularly preferably 1.4301 or 1.4571.

The reaction region is followed according to the invention by an after-treatment unit. Any device which is known to the person skilled in the art and appears to be suitable for improving a certain parameter of the crude product obtained in the reaction can be conceived as the after-treatment unit. For example, a purification or separating device can be provided as the after-treatment unit. Devices which have both a purifying and a separating action are usual in particular. Distillation units, filters, filter presses, sieves, separators, clarification devices or centrifuges, or a combination of two or more of these, are preferably suitable as after-treatment units.

A line for removing a gaseous fluid stream which, for example, can remove by-products with a molecular weight of less than 100 g/mol is furthermore preferably provided on the reactor, this line being connected, if desired, to a pressure reducing unit for applying a reduced pressure. The fluid stream can furthermore be further treated, and for this led over at least one heat exchanger in order to cool the fluid stream. In this context, at least a part of the fluid stream can pass into a liquid phase, which is often collected and led back into the reactor or removed. In this context, at least a part of the fluid stream can pass into a liquid phase, which is often collected and led back into the reactor or removed. This treatment of the fluid stream can be repeated twice or more often. If the fluid stream is led over at least two heat exchangers arranged in series, in the first heat exchanger a part of the fluid stream which differs from that in the at least second heat exchanger can pass into a liquid phase. It is thus possible, if desired, to lead a part of the fluid stream back into the reactor as a liquid phase and to discard another part of the fluid stream. Furthermore, the part of the fluid stream which is to be led back into the reactor as a liquid phase can optionally be divided into two immiscible phases in a separator with the aid of an adjustable removal device. Such a removal device is configured, for example, as an interfacial layer regulator. The first phase can then be passed back into the reactor via a return line. Alternatively, the entire fluid stream can be drained off and e.g. fed to another use, or discarded. The division of the fluid in the separator into two immiscible phases is carried out by appropriate alignment of the interfacial layer regulator. In principle any known embodiment which appears to be suitable to the person skilled in the art is suitable as the interfacial layer regulator.

It is furthermore preferable to collect the fluid stream in a receiver before introduction into the separator, to lead the fluid stream over an additional heat exchanger and to further cool it in this way. At a lower temperature of the fluid stream, a better and faster demixing of at least two immiscible, liquid phases can be observed.

A heat transfer medium is led through each of the heat exchangers already mentioned. In order to effect cooling of the fluid stream, cooling fluids are preferably employed as heat transfer media. Preferably, the highest possible temperature difference is chosen between the fluid stream to be liquefied and the cooling fluid, in order to achieve marked cooling of the fluid stream. Furthermore, it may be entirely desirable to cool the fluid stream in a first step merely to a first temperature at which a part of the fluid stream is liquefied, before a further part of the fluid stream is liquefied in a downstream heat exchanger. It is conceivable that a first heat exchanger is operated with a cooling fluid which, for example, is at 20 or 25 °C, or has a higher temperature, in order to separate off from the fluid stream at least a high-boiling content of the fluid stream which, e.g. has a boiling point in a range of from 80 to 120 °C.

In this connection, a high-boiling content is understood as meaning one or more components of the fluid stream which have a boiling point in a range of from 50 to 150 °C, preferably from 60 to 140 °C, very particularly preferably from 70 to 130 °C. In particular, a high-boiling content is understood as meaning those components which have a boiling point of from 80 to 160 °C, in particular from 90 to 200 °C or more.

According to a further, preferred embodiment, the reactor is connected on its under-side to the delivery pump by fluid-conducting means. In principle, all pumps known to the person skilled in the art which this person considers suitable for carrying out the process according to the invention, taking into account the properties of the liquid to be delivered, which is optionally also in the form of a suspension, dispersion or emulsion, are suitable as the delivery pump. Preferably, a centrifugal, reciprocating, screw, impeller or hose pump can be employed. A centrifugal pump is very particularly preferred.

According to a further preferred embodiment, a delivery line from the delivery pump is connected to an external heat exchanger by fluid-conducting means, the external heat exchanger being connected to the reactor, preferably to the upper side thereof, by fluid-conducting means. The external heat exchanger is connected to the reactor preferably via a return line of not more than 300 cm to 1 cm length, particularly preferably less than 200 cm to 10 cm length, most preferably less than 100 cm to 40 cm length. Particularly preferably, the external heat exchanger is connected directly, preferably via a flange, to the upper side of the reactor.

By the use of the external heat exchanger, the introduction of energy into a delivery stream led via this can be established better both with respect to the duration of the introduction and with respect to the amount of energy, i.e. the heat supplied or removed. This form of introduction of energy renders possible a short duration of the introduction, and therefore little or no change at all to the substance treated in the heat exchanger, in the case of heat-sensitive substances, that is to say those which readily decompose or change. Furthermore, with the use of an external heat exchanger an advantageous ratio of heat transfer area in the heat transfer zone of the heat exchanger to reactor volume can be established.

The delivery stream is preferably led over the heat transfer surface as a film. In this case, the delivery stream has a low height above the heat transfer surface. This arrangement renders possible both a high and a uniform energy transfer rate, so that short energy introduction times are possible compared with other heat transfer arrangements or heat transfer devices. The exposure of the process components to heat in the delivery stream is therefore reduced. Furthermore, undesirable side reactions, e.g. oxidation or polymerization, can likewise be reduced or even avoided.

By suitable choice of the dimensions of the heat transfer surface, in particular of the zone in the flow direction swept over by the film, the volume throughput of the delivery stream and the amount of energy introduced into this can be adapted to the circulation throughput, and thus to the requirements of the process according to the invention. Preferably, a high ratio of heat transfer area in the heat exchanger to volume throughput of the delivery stream is chosen. Furthermore, a ratio of heat transfer area to volume throughput of the delivery stream in a range of from 15 to 1 h/m, particularly preferably from 5 to 1.1 h/m, further preferably from 2 to 1.3 h/m and most preferably from 1.7 to 1.4 h/m is preferred.

For example, the external heat exchanger is configured as a falling film evaporator. In this case, on entry into the heat exchanger the delivery stream is divided and applied as a film to the inner surfaces of tubes connected to the falling film evaporator inlet by fluid-conducting means and preferably arranged side by side, the tube walls forming the heat transfer surfaces. The sum of the individual heat transfer surfaces of the individual tubes forms the heat transfer surface of the falling film evaporator. On passage of the divided delivery stream from the tube into the outflow of the falling film evaporator, the delivery stream is combined again.

The amount of energy per unit volume of the delivery stream which can be transferred in the heat exchanger is determined by the speed of the delivery stream, the distance flowed over in the direction of flow on the heat transfer surface and by the average thickness of the film when sweeping over the heat transfer surface. The thickness of the film means the height of the film over the heat exchanger surface. Preferably, the thickness of the film is in a range of from 2 to 20 %, particularly preferably from 5 to 15 %, and furthermore from 7 to 12 %, in each case based on the internal diameter of the heat transfer surface constructed as tubes.

A further outlet can be positioned on the reactor under-side. The ester A can be removed from the reactor via this, e.g. by a second delivery pump, after the reaction has ended or been discontinued, and fed to a further processing stage, e.g. a filling unit, a heat exchanger, a processing and/or after-treatment unit. Preferably, in the context of that said so far, the under-side of the reactor has an outlet via which both the delivery stream during the reaction and the ester A are led out of the reactor. In order both to be able to lead the process components over an external heat exchanger as a delivery stream during the reaction, and to be able to lead the ester A via the same outlet after the reaction in the reactor, a delivery pump on the outlet of which a distributing device is arranged is preferably provided on the outlet of the reactor. Several connections leading away are provided on this distributing device, at least a first connection being connected by fluid-conducting means to the external heat exchanger and a second connection to a feed to a further processing stage.

According to a further preferred embodiment, a reservoir is provided as a further processing stage between the reaction region and after-treatment unit. This often has at least one storage container and optionally further installations. All embodiments which are known to the person skilled in the art and appear to be suitable are possible as the storage container. Preferably, the storage container has a tank with a stirrer and heating jacket, it being possible for the heating jacket to be arranged on the inside or outside. In such a reservoir, the crude product or the product can be kept for an after-treatment without prolonging the occupancy of the reactor.

The reactor can furthermore have at least one educt reservoir. Any desired installations in which process components can be kept ready before the reaction are conceivable as the educt reservoir. A storage container, a tank, a boiler or a still is preferred. It is likewise possible to provide a storage container connected to a further production plant as the educt reservoir.

According to a further, preferred embodiment, the educt reservoir is connected to the reactor via a line which is led via a preheating stage. All devices known to the person skilled in the art which this person considers suitable for achieving this aim in carrying out the process according to the invention can be employed as the preheating stage. A plate or tube bundle heat exchanger, or a combination of two or more of these, is particularly suitable as the preheating stage. A tube bundle heat exchanger is preferred.

According to a further, preferred embodiment, the educt reservoir is temperature-controllable, for example by a heating jacket or cooling jacket for the educt reservoir. It may be desirable here for a substance which is solid at the ambient temperature to be kept ready above its melting point. If this substance is present in liquid form, simple, often also more accurate metering than e.g. in the case of metering of the solid is possible. Furthermore, by leading substances in closed lines, the risk of exposure and contamination of employees and the environment is avoided.

Preferably, the reactor is furthermore connected to a pressure reducing unit. This is preferably arranged in a fluid-conducting continuation of the heat exchanger or heat exchangers and is connected to the end of the line for removal and/or treatment of the fluid stream. In principle all units for generating a reduced pressure which are known to the person skilled in the art are suitable as the pressure reducing unit, as long as he would consider them taking into account the reactor design.

The present invention also provides a device for carrying out the process according to the invention described above in this application, wherein the device for the preparation of an ester in granule form at least comprises:
a reaction region (110), as described above;
connected by fluid-conducting means to an after-treatment unit (311),
comprising
   - a pump (1) and
   - at least one pressure release device (2),
      wherein the pump (1) and the pressure release device (2) are connected by fluid-conducting means (3),
   - a contact region (4) following the pressure release device (2),
   - an inlet for a cooling fluid (6) arranged in the contact region (4),
   - an outlet for the cooling fluid (7) arranged above the inlet for the cooling fluid (6), and
   - at least one control means(8).

According to a further preferred embodiment, the control means is suitable and intended for controlling the device according to the process described above, control and data lines being provided between the control means and the device.

In principle, any desired pump which is known to the person skilled in the art and appears to be suitable for carrying out the process according to the invention can be chosen as the pump. The following pumps are particularly suitable: generally displacement pumps which guarantee a fluid pressure as far as possible without variation, such as a screw spindle pump, centrifugal pump or gear pump, a centrifugal pump or a screw spindle pump being particularly preferred.

According to a further preferred embodiment, the material of the spindle of the spindle pump, or of the centrifuge of the centrifugal pump, is in each case harder than the pump housing. According to a further preferred embodiment, the pump can be heated, particularly preferably in a range of from 20 to 200 °C, or from 50 to 150 °C. Such a pump is preferably employed in fluid streams with slightly abrasive or abrasive substances. According to a further preferred embodiment, the pump has a delivery rate of from 2 to 30 m³/h, particularly preferably from 4 to 20 m³/h, or 5 to 15 m³/h.

All pressure release devices which are known to the person skilled in the art and appear to be suitable for carrying out the process according to the invention are possible as pressure release devices. Nozzles, above all one-component nozzles and/or hollow cone nozzles, are suitable in particular as the pressure release device. According to a particularly preferred embodiment, pressure release devices which can be operated under a pressure of from 1 to 100 bar, further preferably 1 to 50 bar, or 2 to 30 bar are chosen. The pressure release devices, in particular as a nozzle, can furthermore be characterized by further structural elements, such as, for example, a rotary insert. A rotary insert generates shearing forces or shear flows, or both, in the material flowing over it. The angle of the spray cone at the outflow which leads into the contact region and the particle size distribution of the released fluid stream in the contact region can thereby be adapted.

According to a further preferred embodiment, two or more pressure release devices are operated in a parallel arrangement in order to increase the throughput of fluid or in order to achieve a more uniform distribution of the fluid in the contact region, or both.

The outflow of the pressure release device leads into a contact region in which a released fluid stream can be cooled, which thereby forms a particle stream under consolidation. Depending on the chemical composition, the temperature of the fluid stream, and the temperature of the counter-current, the person skilled in the art can determine the length of the contact region necessary for the preparation of an ester in granule form without difficulties.

At the end of the contact region is an outlet for removing the particle stream. In principle all devices for closing and opening the outlet which are known to the person skilled in the art and appear to be suitable are suitable as the outlet. In the simplest case, this can be a door. A sluice is particularly preferably employed as the device for closing and opening the outlet. Such devices are also suitable for sluicing out substances if there is a pressure difference at the sluice. Cellular wheel sluices are particularly preferably employed. By means of such a sluice, the particle stream can be removed continuously from the contact region, without noticeable pressure losses occurring in the contact region. A further aspect in connection with cellular wheel sluices is an improved work safety for employees close to the outlet.

One or more inlets through which a cooling fluid can be led are furthermore located on the end of the contact region with the outlet. Preferably, the contact region has several inlets, which make a uniform supply of the cooling fluid possible. Furthermore, one or more removal lines for removal of a cooling fluid are located on the side of the contact region adjacent to the outlet of the pressure release device. The cooling fluid is preferably present in gaseous form.

According to a further preferred embodiment, the contact region can be operated with a reduced pressure. To generate such a reduced pressure, a gas conveyor can be arranged on the one or the several removal lines for the cooling fluid. In principle all apparatuses for conveying gas which are known to the person skilled in the art and appear to be suitable are suitable as the gas conveyor. Ventilators, fans are suitable in particular for this.

According to a further preferred embodiment, the gas conveyor has a throughput in a range of from 10,000 to 60,000 m³/h, in particular from 15,000 to 30,000 m³/h. The throughput is determined with the aid of a vane anemometer after exit of the cooling fluid from the contact region, before entry into the gas conveyor.

The device according to the invention furthermore has several sensors. The amount of substance passed through is preferably determined by means of a flow meter arranged after the compressor, for example a mass flow meter. The temperature of the cooling fluid removed can furthermore be determined before the gas conveyor. Furthermore, at least the values mentioned in the following are determined by means of one or more suitable sensors at the pressure release device before the release: the temperature, the viscosity and the throughput, in each case of the fluid to be released. These values are recorded continuously during operation of the device and are passed on to the control means. This monitors the maintaining of given values, in particular the ratios given above with the formulae (I) to (III), and regulates the pump output of the pump accordingly, so that the particular characteristic values I₁, I₂ and I₃ specified by the production are maintained. This is achieved if the deviation of the actual characteristic value from the intended characteristic value is less than 10 % for more than 50 %, preferably more than 75 %, or more than 90 % of the time in which an ester in granule form is produced.

The present invention also provides a process for the preparation of an ester, wherein the device described above is employed.

An embodiment, which also contains optional features and is in no way intended to represent a limitation of that said so far is explained further by way of example in the following with the aid of drawings.

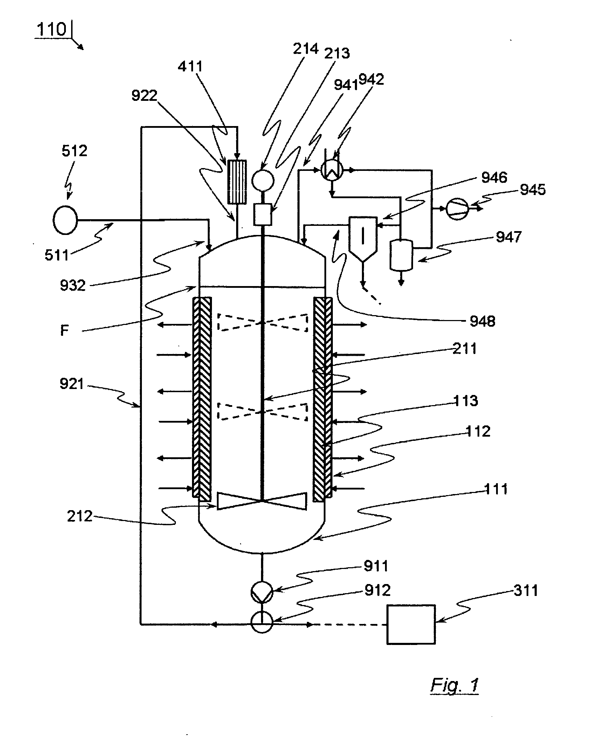

Figure 1 shows a reaction region 110 with a reactor 111 with various installations which is suitable and preferred for carrying out the process according to the invention. The reactor 111 has on the reactor wall an external heating jacket 112. This is divided into three sections, which can be controlled separately. In the middle of the reactor, along its vertical axis, a stirrer 211 with stirrer tiers 212 is arranged. The stirrer 211 is driven via a transmission 213 with a motor 214. Baffles 113 can be arranged on the reactor wall. On the upper side of the reactor 111, an external heat exchanger 411 is arranged via a connection 922, which can be configured as a return line or as a flange. Preferably, the external heat exchanger 411 is configured as a falling film evaporator. On the under-side of the reactor 111 is an outlet with a shut-off valve, which is connected to a delivery pump 911. A distributing device 912, e.g. a multi-way valve, is attached to the outlet of the delivery pump. From the distributing device 921, a return line 921 leads to the external heat exchanger 411. A second line leads from the distributing device 912 to an after-treatment unit 311. The filling level line F represents the position of the interface between the space underneath the interface taken up by the filling volume and the gas space above this.

On the upper side of the reactor 111, a feed line 511 is attached, which is connected to one or more educt reservoirs containing process components. Furthermore, a line 941 for removing a fluid stream leads from the upper side of the reactor 111 to the heat exchanger 942. This line is connected to a receiver 947 and a separator 946. A condensate of the heat exchanger 942 can be fed either directly, or via the receiver 947, to the separator 946. The separator 946 has an interfacial layer regulator, from which a return line 948 leads to the reactor 111. The separator 946 and the receiver 947 can likewise be emptied by an outlet in each case arranged on the under-side. A reduced pressure can be generated by a pressure reducing unit 945 via a line connected to the receiver 947.

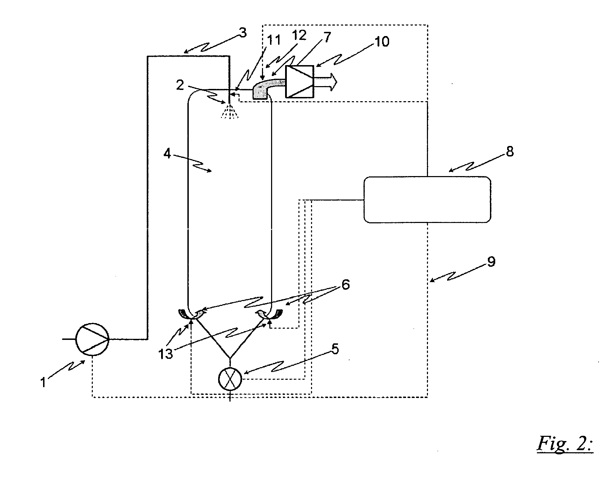

Fig. 2 shows a device with various installations which are suitable for carrying out the process according to the invention for the preparation of the ester in granule form. The contact region 4 is aligned in the direction of the earth's gravity force vector, a pressure release device 2 being installed at the upper end of the contact region 4. A sluice 5 is arranged at the other end of the contact region 4. The contact region 4 narrows towards the sluice 5. The outlet of the sluice 5 leads away from the device for preparation of the ester in granule form to a further processing unit or to packing of the ester in granule form.

The contact region 4, which is preferably a part of a tank or of a spray or trickle tower, is demarcated on its upper side by the opening of the pressure release device 2, the pressure release device being connected by fluid-conducting means to the line 3. At the start of the line 3 is located a pump 1, the outflow from the pump 1 being connected to the line 3, and the pump 1 being connected from its inlet to an earlier after-treatment device or a reactor, e.g. the reactor 111 from Fig. 1. A measurement point 11, which preferably comprises three sensors, is provided at the incident flow of the pressure release device. These determine the temperature *T_{1,E}*, the pressure of the fluid before the release *p_{E},* and the viscosity η*_{E}*.

At the lower end of the contact region 4 are located one or more inlets 6 with a temperature sensor 13, the inlets 6 serving to feed in a cooling fluid. The temperature sensor determines the entry temperature of the cooling fluid in the measurement point T_{1,CF}. At the upper end of the contact region 4 is moreover located an outlet 7 with a temperature sensor 12, the temperature sensor 7 reading the measurement point T_{2,CF} 12. In the outflow of the outlet 7 a gas conveyor 10 is installed with a fluid-conducting connection, the incident flow of the gas conveyor 10 being in a fluid-conducting connection with the contact region 4 via the outlet 12 with the temperature sensor 7.

The measurement points 11, 12 and 13 are connected to the control means 8 via data lines. The control means 8 is furthermore connected to the pump 1 and the sluice 5 via control and data lines.

The present invention also provides a process for the preparation of a thermoplastic composition comprising
a1) a thermoplastic polymer,
b1) an additive, and
c1) optionally further additives,
comprising the process steps:
i) provision of a thermoplastic polymer or of a precursor of a thermoplastic polymer or both;
ii) provision of an additive comprising an ester obtainable by the process according to the invention described above by reaction of at least one alcohol component and at least one carboxylic acid component, the device according to the invention described above preferably being employed;
iii) optionally provision of further additives,
iv) mixing of components i), ii) and optionally iii).

Those esters and further additives which have already been mentioned above as preferred esters and further additives in connection with the processes according to the invention for the preparation of an ester are preferred as esters and further additives.

In a preferred embodiment, the additive comprises a preferably at least partly hardened ester from a carboxylic acid component and an alcohol component with one or more hydroxyl groups.

"Hardened esters" in the present case are understood as meaning esters in which the carboxylic acid components are derived from a carboxylic acid containing one or more double bonds. These double bonds can be at least partly or completely removed by hydrogenation. If not all of the double bonds of the carboxylic acid have been removed, a partly hardened ester is referred to, preferably at least 50 mol% and particularly preferably at least 70 mol% of the double bonds of the carboxylic acid having been hydrogenated, which can be determined, for example, by NMR spectroscopy or by determining the iodine number.

The term "thermoplastic polymer" is understood as meaning plastics which can be (thermo)formed (plastically) in a temperature range which is elevated with respect to room temperature. This operation is reversible and can be repeated by cooling and reheating into the molten state as often as desired, unless thermal decomposition of the material starts due to overheating.

Possible thermoplastic polymers which the composition according to the invention can contain are, in particular, thermoplastic polyurethanes, thermoplastic polyesters, thermoplastic polyamides, thermoplastic polyolefins, thermoplastic polyvinyl esters, thermoplastic polyethers, thermoplastic polystyrenes, thermoplastic polyimides, thermoplastic sulphur polymers, thermoplastic polyacetals, thermoplastic fluorinated plastics, thermoplastic styrene/olefin copolymers, thermoplastic polyacrylates, thermoplastic ethylene/vinyl acetate copolymers or mixtures of two or more of the abovementioned thermoplastic polymers.

It is preferable according to the invention for the thermoplastic polymer to be based on thermoplastic polyesters to the extent of more than 90 wt.%, particularly preferably to the extent of more than 95 wt.%, moreover still more preferably to the extent of at least 99 wt.% and most preferably to the extent of 100 wt.%, in each case based on the total weight of the thermoplastic polymer. The term "polyester" as used herein includes in particular polymers which have been obtained by a polycondensation reaction between a polycarboxylic acid and a polyol (so-called "AA//BB polyesters") or by a polycondensation reaction of a hydroxycarboxylic acid or by ring-opening polymerization of a cyclic ether (so-called "AB polyesters"). In one embodiment according to the invention, polycarbonates which are obtainable e.g. by reaction of phosgene with diols are excluded from the term "polyester" used according to the invention.

In principle, all the thermoplastic polyesters and copolyesters currently known can be used as component a1) in the thermoplastic composition according to the invention. Examples of such polyesters include linear polyesters which have been prepared via a condensation reaction of at least one polycarboxylic acid, preferably a dicarboxylic acid (dibasic acid) or an ester-forming derivative thereof, and at least one polyol, preferably a dihydric alcohol (diol).

It is furthermore conceivable to prepare polyesters which have a degree of branching or crosslinking which is not equal to zero, that is to say are not linear.

In this connection, the degree of branching is the mean, over the sum of all the polyester molecules, of the ratio of the number of branching monomer units to the total number of all the monomer units per polyester molecule. The degree of branching is in a range of from 0.01 to 50 wt.%, preferably from 0.05 to 30 wt.%, further preferably from 0.1 to 20 wt.%, particularly preferably from 0.5 or 1 to 10 wt.% and most preferably between 3 and 7 wt.%, based on the sum of all the thermoplastic polyester molecules. If polyesters which are not exclusively linear but are branched to at least a small proportion, e.g. between 2 and 8 wt.%, are employed as the thermoplastic polyester, an adaptation of the physical properties of the thermoplastic composition, for example a reduction of the viscosity, can be established.

In this connection, the degree of crosslinking is the mean, over the sum of all the polyester molecules, of the ratio of the number of crosslinking monomer units to the total number of all the monomer units per polyester molecule. In this connection, the degree of crosslinking is in a range of from 0.001 to 3 wt.%, preferably 0.005 to 1 wt.%, particularly preferably 0.01 to 0.5 wt.% and most preferably 0.05 to 0.1 wt.%, based on the sum of all the thermoplastic polyester molecules. At these low degrees of crosslinking, the thermoplastic properties of the molecules are retained.

The preferably difunctional acid and the preferably difunctional diol can both be either aliphatic or aromatic, aromatic and partly aromatic polyesters being particularly preferred as thermoplastic moulding materials because of their high softening points and stability to hydrolysis. In the case of aromatic polyesters, between 80 and 100 % of all the ester linkages are added on to the aromatic rings.

These thermoplastic moulding materials can be semicrystalline and even show liquid crystal properties or be amorphous. According to the invention, partly aromatic polyesters which have been obtained from at least one aromatic dicarboxylic acid or an ester-forming derivative thereof and at least one aliphatic diol are particularly preferred thermoplastic polyesters. Examples of suitable aromatic dicarboxylic acids include terephthalic acid, 1,4-naphthalenedicarboxylic acid or 4,4'-biphenyldicarboxylic acid. Examples of suitable aliphatic diols include alkylene diols, specifically those which contain 2 to 6 C atoms, preferably 2 to 4 C atoms, where ethylene glycol, propylene diols and butylene diols are to be mentioned in particular here. Ethylene glycol, 1,3-propylenediol or 1,4-butylenediol are preferably used as the polyol or diol component for the preparation of the thermoplastic polyesters contained as component a) in the composition according to the invention. Thermoplastic polyesters which are obtainable by reaction of a dicarboxylic acid with a diol and are particularly preferred according to the invention include, in particular, polyalkylene terephthalates, for example polyethylene terephthalate (PET), polypropylene terephthalate (PPT) or polybutylene terephthalate (PBT), polyalkylene naphthalates, for example polyethylene naphthalate (PEN) or polybutylene naphthalate (PBN), polyalkylene dibenzoates, for example polyethylene dibenzoate, and mixtures of at least two of these thermoplastic polyesters.

The partly aromatic polyesters described above can optionally contain a small amount of units which originate from other dicarboxylic acids, for example isophthalic acid, or other diols, such as cyclohexanedimethanol, which in general reduces the melting point of the polyester. A specific group of partly aromatic polyesters are so-called segmented or block copolyesters, which in addition to the abovementioned polyester segments (also called "hard segments"), contain so-called "soft segments". These soft segments originate from a flexible polymer; that is to say one with amorphous contents with a low glass transition temperature (T_{g}) and low rigidity to the extent of 60 to 100 wt.%, preferably more than 70 and still more preferably more than 80 wt.% to 100 wt.%, based on the total weight of the polymer. This flexible polymer has reactive end groups, preferably two hydroxyl groups. Preferably, the glass transition temperature of these "soft segments" is below 0 °C, particularly preferably below -20 °C and most preferably below -40 °C. In principle, several different polymers can be used as the soft segment. Suitable examples of "soft segments" are aliphatic polyethers, aliphatic polyesters or aliphatic polycarbonates. The molecular weight of the soft segments can vary within wide limits, but is preferably between 400 and 6,000 g/mol.

In addition to the abovementioned linear polyesters which are obtainable via a polycondensation reaction of at least one polycarboxylic acid or an ester-forming derivative thereof and at least one polyol, the thermoplastic composition according to the invention can also contain thermoplastic polyesters which are obtainable by a polycondensation reaction of short-chain hydroxycarboxylic acids or by a ring-opening reaction of cyclic esters.

Examples of suitable short-chain hydroxycarboxylic acids which can be employed for the preparation of thermoplastic polymers include in particular L-lactic acid, D-lactic acid, DL-lactic acid, glycolic acid, 3-hydroxybutyric acid, 4-hydroxybutyric acid, 4-hydroxyvaleric acid, 5-hydroxyvaleric acid, 6-hydroxycaproic acid and mixtures of these hydroxycarboxylic acids. Examples of suitable cyclic esters include in particular glycolide (a dimer of glycolic acid) and ε-caprolactone (a cyclic ester of 6-hydroxycaproic acid).

The preparation of the thermoplastic polyesters described above is also described, inter alia, in "Encyclopedia of Polymer Science and Engineering", volume 12, pages 1 to 75 and pages 217 to 256; John Wiley & Sons (1988) and also in "Ullmann's Encyclopedia of Industrial Chemistry", volume A21, pages 227 to 251, VCH Publishers Inc. (1992). Thermoplastic polymers which are preferred according to the invention are polyethylene terephthalate (PET), polybutylene terephthalate (PBT) and polylactic acid (PLA), it being possible for a preferred embodiment of a thermoplastic composition of the present invention to contain each of this polymers in itself to the extent of more than 50 wt.%, preferably more than 75 wt.% and particularly preferably more than 90 wt.%, in each case based on the thermoplastic composition.

Components a1), b1) and optionally c1) are first provided in process steps i), ii) and optionally iii). The mixing of components i), ii) and optionally iii) is then carried out in process step iv) of the process according to the invention.

In this context, the mixing of components a1), b1) and optionally c1) can be carried out utilizing known techniques. Thus, the mixing can be, for example, a dry mixing operation, in which the various components are mixed at below the melt processing temperature of the thermoplastic polymer, or a melt mixing process, in which the components are optionally premixed, and mixed at the melt processing temperatures of the thermoplastic polymer. The melt mixing processes include, preferably, the melt kneading process, which can be realized, for example, by continuous melt kneading using a single-screw kneading machine, a twin-screw kneading machine of the toothed-same direction of rotation type, toothed-opposite direction of rotation type, nontoothed-same direction of rotation type, nontoothed-opposite direction of rotation type, or other types, or by batch melt kneading using a roller kneading machine, a Banbury kneading machine or similar. A combination of a dry mixing process and a melt mixing process is furthermore conceivable.

The sequence and the nature and manner of the addition of the individual components a1), b1) and optionally c1) into the mixing device is furthermore in principle not critical. Thus, for example, the thermoplastic polymer and optionally the additional substances can first be initially introduced into the mixing device and the additive only then added. It is also conceivable for the additive or a part of the additive first to be mixed with one or more other components of the thermoplastic composition according to the invention, for example with one or more additional substances, and then either for this mixture to be added to the thermoplastic polymer already in the mixing device, or for this mixture first to be initially introduced into the mixing device and the thermoplastic polymer only then to be added.

In further embodiments of the process according to the invention for the preparation of a thermoplastic composition, mixing is carried out in accordance with at least one of the following measures:
M1) at the glass transition temperature of the thermoplastic polymer or at a temperature above the glass transition temperature of the thermoplastic polymer;
M2) where the additive is more liquid than the thermoplastic polymer; or
M3) where at least a part of the additive is added to the precursor of the thermoplastic polymer.

Embodiments according to the invention furthermore include combining of two or more of the above measures. The following combinations of measures represented with the aid of the combinations of figures thus result specifically as embodiments: M1M2, M1M3, M2M3 and M1M2M3.

According to a preferred embodiment M1 of the process according to the invention, the mixing of components i), ii) and optionally iii) is carried out in process step iv) of the process according to the invention by a melt mixing process. In this connection it is preferable in particular for the mixing in process step iv) to be carried out at the glass transition temperature of the thermoplastic polymer or at a temperature above the glass transition temperature of the thermoplastic polymer. In this connection, it is particularly preferable for the mixing to be carried out at a temperature in a range of from 5 °C below the glass transition temperature (T_{g}) to 200 °C above the glass transition temperature of the thermoplastic polymer employed, particularly preferably at a temperature in a range of from 1 °C below the glass transition temperature (T_{g}) to 180 °C above the glass transition temperature of the thermoplastic polymer employed and most preferably at a temperature in a range of from 1 °C above the glass transition temperature (T_{g}) to 150 °C above the glass transition temperature of the thermoplastic polymer employed, the upper limit of the temperature range being essentially limited, however, by the decomposition temperature of the thermoplastic polymer employed. Embodiments according to the invention furthermore include mixing at temperatures in a range of from 10 to 180 °C and preferably 50 to 150 °C above the glass transition temperature of the thermoplastic polymer employed.

In embodiment M2 according to the invention, in which the additive is more liquid than the thermoplastic polymer, it is preferable for the additive to be employed at a temperature at which this is liquid and the thermoplastic polymer is not yet liquid. The temperature of the thermoplastic polymer here is preferably below the glass transition temperature of this polymer. It is thus preferable for the melting temperature of the additive and the glass transition temperature of the thermoplastic polymer to differ by at least 5 °C, preferably at least 10 °C and particularly preferably at least 30 °C. In this embodiment and also generally, it is furthermore preferable for the thermoplastic polymer to be employed as granules. In general, all forms of granules known to the person skilled in the art with a spherical or cylindrical spatial shape are also possible in the present case. The granule size, determined by means of sieve analysis, is in a range of from 0.01 to 5 cm, and preferably in a range of from 0.1 to 4 cm for at least 70 wt.% of the granule particles. By the procedure according to this embodiment, the surfaces of the granule particles can be at least partly coated with the additive according to the invention, so that at least partly coated thermoplastic polymer granules are obtained. This allows a distribution of the additive according to the invention in the thermoplastic composition which is as homogeneous as possible, especially if this is made up as a formulation for the extrusion taking place later.

In embodiment M3 according to the invention, in which the additive is added to the precursor of the thermoplastic polymer, additive in the liquid form and also in the solid form are possible. Possible precursors of the thermoplastic polymer are in principle all the precursors before the thermoplastic polymer is obtained which are known to the person skilled in the art. These include, in particular, precursors which have a lower molecular weight than the final thermoplastic polymer. It is preferable here for the molecular weight of the precursor to differ from that of the finished thermoplastic polymer by a factor of at least 1.1, preferably at least 1.5 and particularly preferably at least by a factor of 2. In addition to the monomers and oligomers, which preferably comprise 2 to 100 monomers, employed for the preparation of the thermoplastic polymer, a prepolymer which is polymerized completely, usually by heat treatment, to give the finished thermoplastic polymer is included, especially in the case of polycondensates. The prepolymer is preferably based on more than 100 monomers as recurring units, the number of the monomers as recurring units and therefore the final molecular weight of the finished thermoplastic polymer not being reached. It is therefore particularly preferable for the additive according to the invention in each case to be added to the monomers, oligomers or the prepolymer or at least two of these. By this means, in addition to a homogeneous distribution of the additive according to the invention, incorporation of the additive by chemical bonds with the thermoplastic polymer is also achieved, usually by the conditions prevailing during the polymerization or complete polymerization.

If the heated composition obtained in process step iv) in the case of a melt mixing process is not fed directly to the production of shaped articles, the process can also additionally include the further process step v):
v) cooling of the thermoplastic composition, preferably to a temperature in a range of from 20 to 30 °C, particularly preferably to room temperature.

The thermoplastic composition which has been obtained in process step iv) can furthermore be fed to a granulation before, during or also after carrying out process step v), but optionally also after process step iv) and without carrying out process step v).

Furthermore, in addition to the thermoplastic polymer (component a1) and the additive (component b1), the thermoplastic composition according to the invention can optionally also contain further additives (component c1). The further additives include in particular impact modifiers, filler materials, reinforcing agents, flame retardant compounds, heat and UV stabilizers, antioxidants, other processing auxiliaries, nucleating agents, dyestuffs and antidripping agents. Examples of suitable impact modifiers, filler materials, reinforcing agents and flame retardant compounds are to be found, inter alia, in US 2005/0234171 A1.

It is furthermore preferable in connection with the process according to the invention for components a1) to c1) to be mixed with one another in relative amounts such that the thermoplastic composition obtained by mixing components a1) to c1) contains
a11) at least 40 to 99.99 wt.%, particularly preferably 50 to 99.8 wt.% and most preferably 60 to 99.6 wt.% of the thermoplastic polymer,
b11) 0.01 to 60 wt.%, particularly preferably 0.1 to 40 wt.% and most preferably 0.2 to 5 wt.% of the additive and
c11) 0 to 20 wt.%, particularly preferably 0.1 to 10 wt.% and most preferably 0.2 to 5 wt.% of the further additives
in each case based on the total weight of the thermoplastic composition, wherein the sum of components a11) to c11) is 100 wt.%.

In another process embodiment according to the invention, it is preferable for components a12) to d12) to be mixed with one another in relative amounts such that the thermoplastic composition obtained by mixing components a12) to d12) contains
a12) 1 to 69.99 wt.%, particularly preferably 1.5 to 49.8 wt.% and most preferably 2 to 19.6 wt.% of the thermoplastic polymer,
b12) 0.01 to 20 wt.%, particularly preferably 0.1 to 10 wt.% and most preferably 0.2 to 5 wt.% of the additive,
c12) at least 10 wt.%, preferably at least 20 wt.% and particularly preferably at least 30 wt.% of a biodegradable filler component and
d12) 0 to 20 wt.%, particularly preferably 0.1 to 10 wt.% and most preferably 0.2 to 5 wt.% of the further additives
in each case based on the total weight of the thermoplastic composition, wherein the sum of components a12) to d12) is 100 wt.%. Possible biodegradable filler components are in principle all those which are known to the person skilled in the art and appear to be suitable. These include, in particular, mono- and polysugars, such as starch and starch derivatives, cellulose and cellulose derivatives, hemp, jute, bast, rush, reed, in particular reed flour, and other substances obtained from plants or a combination of at least two of these. In the context of this embodiment, it is furthermore preferable for the thermoplastic polymer to be based on a monomer which can be generated from renewable raw materials, such as lactic acid, to the extent of at least 10 wt.%, preferably to the extent of at least 50 wt.% and particularly preferably to the extent of at least 75 wt.%, in each case based on the thermoplastic polymer. This thermoplastic composition is suitable in particular for biodegradable non-returnable and disposable articles, such as utensils or cutlery.

It is moreover preferable according to the invention for a saturated, α-olefinic oligomer of at least one C₆-C₁₈-α-olefin to be employed in the course of the process according to the invention for the preparation of a thermoplastic composition in at most an amount such that the thermoplastic composition obtained by mixing components a1) to c1) contains less than 0.001 wt.%, particularly preferably less than 0.0005 wt.% and most preferably less than 0.0001 wt.% of the saturated, α-olefinic oligomer.

A contribution towards achieving the abovementioned objects is furthermore made by the thermoplastic composition obtainable by the process described above. Here and generally, it is preferable for the thermoplastic composition to have a yellow value of less than 6.64, preferably less than 6, particularly preferably less than 5 and furthermore preferably less than 4 and moreover preferably less than 3. The yellow value is often less than 2, or than 1. In the ideal case it is 0, but often more than 0.1 or 0.2.

The present invention also provides a process for the production of a shaped article, comprising the process steps:
I) provision of a thermoplastic composition obtainable by the process described above for the preparation of a thermoplastic composition;
II) heating of the thermoplastic composition to the glass transition temperature of the thermoplastic polymer or to a temperature above the glass transition temperature of the thermoplastic polymer;
III) production of a shaped article from the heated thermoplastic composition prepared in process step II).

In step I) of the process according to the invention for the production of a shaped article, a thermoplastic composition according to the invention is first provided, this provision preferably being carried out by a process according to the process described above for the preparation of a thermoplastic composition.

In process step II), the thermoplastic composition is then heated to the glass transition temperature of the thermoplastic polymer or to a temperature above the glass transition temperature of the thermoplastic polymer. In this connection, it is in turn preferable for the heating of the thermoplastic composition to be carried out to a temperature in a range of from 5 °C below the glass transition temperature (T_{g}) to 100 °C above the glass transition temperature of the thermoplastic polymer employed, particularly preferably to a temperature in a range of from 1 °C below the glass transition temperature (T_{g}) to 50 °C above the glass transition temperature of the thermoplastic polymer employed and most preferably to a temperature in a range of from 1 °C above the glass transition temperature (T_{g}) to 20 °C above the glass transition temperature of the thermoplastic polymer employed, here also, however the upper limit of the temperature range being essentially limited by the decomposition temperature of the thermoplastic polymer employed.

In principle, process steps I) and II) can be carried out simultaneously or in succession. It is appropriate to carry out process steps I) and II) simultaneously, for example, if the thermoplastic composition is prepared by means of a melt mixing process. Where appropriate, it may be advantageous here to convert the composition prepared by the melt mixing process directly into a shaped article. It is appropriate to carry out process steps I) and II) successively, for example, if the thermoplastic composition is prepared by means of a dry mixing process or if the thermoplastic composition is indeed prepared by means of a melt mixing process, but is not subjected to the formation of a shaped article directly after the preparation, but rather is first cooled according to process step v).

In process step III) of the process according to the invention for the production of a shaped article, a shaped article is produced from the heated thermoplastic composition prepared in process step II). Possible processes for the production of a shaped article are, in particular, injection moulding, extrusion moulding, compression moulding, layer moulding, laminating moulding, blow moulding, vacuum moulding and transfer moulding, injection moulding being particularly preferred.

Furthermore, in a preferred embodiment of the process according to the invention for the production of a thermoplastic shaped article, in at least one further process step IV) at least a part region of the shaped article obtained in process step III) serves as a shaped article blank and is reduced in its mass cross-section compared with process step III). The mass cross-section is the cross-section of a region of the shaped article made solidly from the thermoplastic moulding composition according to the invention. For example, in containers or vessels, the mass cross-section is the thickness of a wall of these containers or vessels. In the case of shaped articles which are rather thread- or strand-like in construction, the mass cross-section is the thickness of these threads or strands. In the case of rather planar structures, such as sheets, layers, webs, films or foils, the mass cross-section is the thickness of these planar structures. For the reduction in the mass cross-section, in principle all the methods known to the person skilled in the art and suitable for this are possible. These include, for example, stretching in one or two directions, drawing in one or two directions, centrifugation or blowing, each of which is preferably carried out at elevated temperatures at which the thermoplastic composition according to the invention is so soft or even liquid that stretching, drawing, centrifugation or blowing can be carried out. The part region in which the reduction in cross-section is effected preferably makes up at least 50 % and particularly preferably at least 80 % of the shaped article obtained in step III). Stretching or drawing are generally carried out if a fibre is to be obtained from the shaped article obtained in step III). For the production of films, on the one hand drawing or stretching in one or more dimensions can be carried out. Thus, the web running out of an extruder can be drawn on to a roll at a higher speed compared with the exit speed from the extruder. On the other hand, if a container or vessel is to be obtained, apart from stretching, drawing and centrifugation, blowing is chiefly employed in step IV). In this, the reduction in mass cross-section is effected by applying a gas pressure. The gas pressure is generally chosen such that the thermoplastic composition, which is usually heated at least to the glass transition temperature, of the shaped article obtained in step III) can be extended. The extending is as a rule limited by using a mould having the final shape of the shaped article. In addition to containers, such as freezer boxes, dishes and packaging for foodstuffs, such as fruit, vegetables or meat, as well as medicaments as tablets, capsules, suppositories or powders, vessels for liquids can also be produced in this way. As well as for liquids of the cosmetics or pharmaceuticals industry, these vessels for liquids can also be used in the foodstuffs industry, preferably in the drinks industry, as reusable vessels, such as PET or PLA bottles. It is furthermore possible for two or more of process steps I) to IV) to be supplemented by further process steps and/or to at least overlap in time. This applies in particular to process steps III) and IV).

In addition to bottles, other shaped articles can furthermore also be produced according to the invention. These include disposable and reusable vessels, such as plates, dishes, pots or beakers, and cutlery, such as knives, forks or spoons. The biodegradable thermoplastic compositions according to the invention are particularly suitable for these uses.

The present invention also provides a process for the production of a packed product, comprising the process steps:
a3) provision of a product and a shaped article, in particular a film, the shaped article being obtainable by the process described above;
b3) at least partial surrounding of the product with the shaped article.

The product provided in process step a) is preferably a pharmaceutical, a body care composition, an agricultural auxiliary substance, an adhesive, a building material, a dyestuff or a foodstuff.

The at least partial surrounding of the product can be carried out, for example, by the process described in DE-A-103 56 769.

The present invention also provides a process for the production of an at least partly coated object, comprising the process steps:
a4) provision of a coating composition comprising at least 10 wt.%, based on the coating composition, of an ester or a thermoplastic composition obtainable by the particular process described above, and of a solid substrate;
b4) mixing of the coating composition and the substrate, the coating composition being at least partly liquid.

The production of the at least partly coated object with the coating composition can be carried out, for example, by a procedure in which the coating composition and the object to be coated are mixed with one another in suitable mixing devices, the Patterson-Kelly mixer, DRAIS turbulence mixer, Lödige mixer, Ruberg mixer, screw mixers, plate mixers and fluidized bed mixers being possible in particular as mixing devices. Should the coating composition not be liquid under the mixing conditions, this component is to be heated to a temperature above the melting temperature of the coating composition before or during the mixing with the object to be coated. In addition to the use of the mixing devices described above, the production of the at least partly coated object can also be carried out by a procedure in which, for example, the object to be coated is initially introduced into a fluidized bed mixer and the coating composition is sprayed in liquid form on to the object to be coated.

The present invention also provides a further processing product comprising as an additive an ester which can be prepared by the process according to the invention described above, and moreover at least one functional component chosen from the group consisting of thermoplastic polymer, enzyme, curing agent of an adhesive, paraffin, oil, colouring agent, hair or skin care substance, polymer dispersion, lime mud, lubricant or emulsifier, or a combination of two or more of these.

The present invention also provides the use of an ester obtainable by the process according to the invention described above as an additive in a composition which is chosen from the group consisting of thermoplastic composition, detergent, adhesive, defoamer, lubricant formulation, lacquer, paint, cosmetic formulation, soil compacting agent, drilling mud, hydraulic oil or dispersion.

According to a further preferred embodiment, the ester is employed as an additive in a composition comprising as a functional component
α) a thermoplastic polymer, the composition being a thermoplastic composition;
β) an enzyme, the composition being a detergent;
γ) a curing agent of an adhesive, the composition being an adhesive;
δ) a paraffin, the composition being a defoamer;
ε) an oil, the composition being a lubricant formulation;
ζ) a colouring agent, the composition being a lacquer or a paint; or
η) a hair or skin care substance, the composition being a cosmetic formulation,
θ) a polymer dispersion, the composition being a soil compacting agent,
τ) a lime mud, the composition being a drilling mud,
κ) a lubricant, the composition being a hydraulic oil,
λ) an emulsifier, the composition being a thermoplastic composition or a dispersion.

Preferably, the additive is employed in an amount in a range of from 0.001 to 40 wt.%, particularly preferably in a range of from 0.01 to 20, very particularly preferably from 0.1 to 10 wt.% and particularly preferably in a range of from 0.5, 1 or 2 to 5, 6, 7 or 8 wt.%, based on the composition.

The invention is now explained in more detail with the aid of non-limiting examples.

### Measurement methods

Unless expressly stated otherwise, all the measurements are carried out in accordance with the relevant ISO standards. Unless specified otherwise there, a temperature of 23 °C, an atmospheric pressure of 1 bar and a relative atmospheric humidity of 50 % was chosen.

### Composition of a mixture of several carboxylic acid components

Mixtures of several carboxylic acid components such as are present, for example, in technical grade oleic acid can be determined by means of gas chromatography (GC) or high pressure liquid chromatography (HPLC). The weight contents are stated in wt.%, based on the total weight of the sample supplied.

### Determination of the yellow value

As a measure of the yellow shading/yellowing, the yellow value is determined in accordance with DIN 5033 as the b* value of a sample of the composition to be analysed according to the L*,a*,b* colour system.

### Thermal analysis

The thermal parameters were determined by means of DSC (dynamic heat flow differential calorimetry) on a Mettler Toledo DSC 821. The amount weighed out was 20 - 25 mg in an open crucible. After closing the crucible, the samples prepared in this way were measured over a temperature range of from -100 to +120 °C at a heating rate of 20 K/min against an empty, likewise closed crucible. The sample was kept at +120 °C for 60 seconds and then cooled with the highest possible cooling rate of the apparatus, but at least 15 K/min, to the abovementioned minimum temperature, and kept at this for 2 min before the heating and cooling operation was repeated.

The glass transition temperature and the melting temperature are determined in accordance with DIN 53765.

The degree of crystallization can be determined with the aid of the formula: K = ΔHₘ / ΔHₘ° x 100 %, where K represents the degree of crystallization, ΔHₘ represents the enthalpy of fusion of the sample and ΔHₘ° represents the enthalpy of fusions of the material at a crystallinity of 100 %.

The ΔHₘ° values can be found by the person skilled in the art in the tabular works known to him, e.g. J. Brandrup et al., "Polymer Handbook", 4th ed., John Wiley & Sons.

### Further methods

The following characteristic values are determined in accordance with published standards:

| | | |
|---|---|---|
| Characteristic value | Standard | Comments |
| BET surface area | DIN 66131 | with nitrogen |
| Hydroxyl number (OHN) | DGF C-V 17a | |
| Acid number (AN) | DGF C-V 2 | |
| Saponification number (SN) | DIN 53401 | |
| Pour point | DIN ISO 3016 | |
| Cloud point | DIN ISO 3015 | |
| Glass transition temperature (T_{g}), melting point (Tₘ) | DIN 53765 | see above |
| Density | DIN 51757 | at 20 °C |
| Viscosity | DIN 53015 | at 20 °C |
| Gardner colour number | DIN EN ISO 4630-1 | |
| Lovibond colour number | ISO 15305 | |
| Hazen colour number | DIN ISO 6271 | |
| Thickness of the multifilament [µm] | DIN 53855 Part 1 | |
| Water content | DIN 51777 | [Karl-Fischer method] |

Unless noted otherwise, the raw materials, obtainable under the trade names given, are obtainable from Cognis Oleochemicals Deutschland GmbH, Düsseldorf, or from Sigma-Aldrich Chemie GmbH, Steinheim.

### Examples

### 1. Preparation of esters

The esters shown in the following Table 1 were prepared in an installation which corresponds in diagram form to that shown in Figure 1. The crude product formed in this was then transferred into the installation 311, which is configured as a storage container. From there, a line leads to the installation shown in Figure 2, to which it is connected by fluid-conducting means with the pump 1. The crude product held in the storage container 311 can thus be further processed with the installation shown in Figure 2.

More precisely, for carrying out the experiments components 1, 2 and optionally 3, and optionally further components, were fed according to the mass ratios shown in Table 1 from the storage reservoirs 512 to the reactor 111. The reactor occupancy was between 10,000 and 25,000 kg, and the resulting filling level line F was at 50 to 90 % of the height of the reactor tank.

If the melting point of one component is less than 100 °C, the storage reservoir was configured as a thermostatically controlled tank, the thermostatic control of the therefore liquid component being chosen at a temperature of from about 1 to 5 °C above the melting temperature range of the component present.

If a component, e.g. the catalyst or one of the process components, is introduced into the reactor 111 as a solid, this is effected through a sluice installed on the upper side of the reactor, configured here as a closable, pressure-resistant flap (not shown).

From 1 to 15 kg, depending on the reactor occupancy, if zinc oxalate were then introduced as a catalyst into the reactor 111 via the abovementioned flap. All the components present in the reactor 111 in their entirety are then homogenized with a rotating stirrer provided in the reactor 111, and led in circulation over the heat transfer surface configured as a falling film evaporator 411.

This mixture of components was treated with a elevated temperature (approx. 150 to 240 °C) for between 8 and 30 hours, depending on the choice of components, while stirring. Over the entire time the reactor was operated under reduced pressure (≤ 1,013 mbar) and the reaction was carried out under reflux. The pressure in the reactor was initially 1,000 mbar and was lowered to the desired target vacuum, for example to 10 mbar, in the course of the treatment. Furthermore, the heating of the mixture to the desired temperature and for mixing the substances were boosted by means of pumping in circulation via the delivery pump 911, the distributor 912 and a falling film evaporator 411 from the base of the reactor to the upper side of the reactor and there into the reactor space.

Thereafter, the mixture in the reactor 111 was conveyed via the delivery pump 911 and the distributor 912 into the storage tank 311 and adjusted there to a temperature of approx. 5 °C or more above the melting temperature of the ester prepared.

If desired - in particular for removal of undesirable by-products and/or improving the colour value - the mixture in the storage tank was then mixed with a mixture of 25 kg of kieselguhr (Celite 535, Brenntag N.V., Deerlijk, Belgium) and 5 kg of active charcoal (Norit SA-2, Norit Deutschland GmbH, Kempen, Germany), with vigorous stirring. This mixture was then led and filtered continuously over a filter press (20 chambers, filter surface 13 m³, pressing volume 2501, delivery 3 m³/h) with a circulating pump for one hour.

The following esters were prepared by this procedure:

**Table 1: Overview of the general test instructions, Example 1-6.**

| Ex. no. | Component 1 | Component 2 | Component 3 | Molar ratio of the components |
|---|---|---|---|---|
| 1 | phthalic anhydride | C₁₆₋₁₈-fatty alcohol mixture | - | 1 : 3.4 |
| 2 | C₁₆₋₁₈-fatty acid fraction | C₁₆₋₁₈-fatty alcohol mixture | - | 1 : 1 |
| 3 | C₁₆₋₁₈-fatty acid fraction | pentaerythritol | - | 6.8 : 1 |
| 4 | C₁₆₋₁₈-fatty acid fraction | pentaerythritol | adipic acid | 5 : 1.1 : 1 |
| 5 | C₁₆₋₁₈-fatty acid fraction | pentaerythritol | - | 4 : 1 |
| 6 | C₁₆₋₁₈-fatty acid fraction | pentaerythritol | dipentaerythritol | 85 : 10.5 : 1 |

### 2. Preparation of granules

The esters Ex. no. 1 to 6 prepared in instructions 1 are each in themselves pumped as a melt at a temperature T_{1,E} with a screw spindle pump of the Kral MHGAX 55 type (Kraeutler GmbH & Co., Wüstenau, Austria) under a pressure p_{E} at a delivery rate of FL through a Schlick full cone nozzle (Düsen-Schlick GmbH, Modell 553S) into a spray tower. The viscosity of the melt at temperature T_{1,E} under pressure p_{E} was η_{E}. The atomizing takes place at the Schlick nozzle into the spray tower, air being circulated in the spray tower in counter-current from the bottom upwards at a rate of 15,000 m³. During the granulation of the ester, the air had the temperature T_{1,CF} at the inlet and the temperature T_{2,CF} at the outlet, which is located at the upper end of the spray tower. The pressure p_{C} of air in the spray tower was 1 bar. A pourable powder can be removed in an amount of FL via a cellular wheel sluice installed at the lower end of the spray tower. The production and product parameters are summarized in Table 2 and Table 3. In Examples No. V1 and V2, the same ester as in Example No. 4 was used.

**Table 2: Parameters in the ester granulation for Example 1 to 6**

| Example no. | *T_{m,E}* [°C] | η*_{E}* [mPas] | *T_{1,E}* [°C] | *p_{E}* [bar] | *FL* [kg/h] | *T_{1,CF}* [°C] | *T_{2,CF}* [°C] |
|---|---|---|---|---|---|---|---|
| 1 | 46 | 12 | 50 | 5.0 | 3,200 | -2.0 | 24 |
| 2 | 55 | 7 | 60 | 4.5 | 3,000 | -1.5 | 36 |
| 3 | 62 | 20 | 70 | 7.5 | 3,900 | -1.5 | 36 |
| 4 | 56 | 130 | 80 | 22.0 | 2,500 | -1.0 | 20 |
| 5 | 51 | 31 | 60 | 6.5 | 3,500 | -1.0 | 30 |
| 6 | 65 | 22 | 75 | 12.0 | 3,300 | 2.5 | 33 |
| V1 | 56 | 290 | 57 | 1.4 | 2,500 | -2 | 28 |
| V2 | 56 | 30 | 120 | 86 | 5,000 | -1.0 | 20 |

**Table 3: Product parameters for ester granules from Example 1 to 7**

| Example | Characteristic values of the particle size | | | | |
|---|---|---|---|---|---|
| no. | Mass mean | Particles | Particles | Particles | Particles |
| | [µm] | < 200 µm | > 1,000 µm | ≤ 500 µm | ≥ 500 2 mm |
| | | [wt.%] | [wt.%] | [wt.%] | [wt.%] |
| 1 | 720 | 0.7 | 7.4 | 0.0 | 0.0 |
| 2 | 450 | 7.4 | 0.3 | 0.0 | 0.0 |
| 3 | 600 | 0.53 | 2.4 | 0.0 | 0.1 |
| 4 | 380 | 3.5 | 3.6 | 0.1 | 0.0 |
| 5 | 550 | 4.5 | 5.1 | 0.0 | 0.0 |
| 6 | 500 | 2.5 | 1.9 | 0.0 | 0.0 |
| V1 | Not evaluated because of angel hairing | | | | |
| V2 | Not evaluated because of agglomerates | | | | |

### EXAMPLE 7: Preparation of a thermoplastic composition

6 kg of polyethylene terephthalate (PET SP04 from Catalana de Polimers) was introduced into a 15 kg Henschel mixer. The mixing wall temperature was 40 °C. 0.5 wt.% of the ester prepared in Example 5 was furthermore added as a mould release agent. The material was then granulated on a granulator (ZSK 24Mcc) with a stuffing screw.

### EXAMPLE 8: Production of a shaped article

For production of shaped articles from the thermoplastic composition prepared in Example 7, a fully hydraulic injection moulding machine with a hydraulic closing unit of the Battenfeld HM800/210 type was employed. The maximum closing force is 800 kN, the screw diameter is 25 mm. A mould with a conically tapering, rectangular core was used at the test mould. For determination of the demoulding force, a load cell with a maximum measuring range of 2 kN was attached to the ejector rod. The moulding composition was predried at about 225 °C for about 4 hours. Significantly improved demoulding was observed with the thermoplastic composition according to the invention compared with an additive-free moulding composition.

### List of reference symbols on Figure 1

- Reaction region: 110
- Reactor: 111
- Heating jacket: 112
- Baffle: 113
- Stirrer shaft: 211
- Stirrer blade: 212
- Transmission: 213
- Motor: 214
- After-treatment unit: 311
- External heat exchanger: 411
- Feed line: 511
- Educt reservoir: 512
- Delivery pump: 911
- Distributing device: 912
- Circulation line: 921
- Return line, flange: 922
- Feed of process components: 932
- Vapours line: 941
- Heat exchanger: 942
- Pressure reducing unit: 945
- Separator with interfacial layer: 946
- Receiver: 947
- Return line: 948

- Filling level line: F

### List of reference symbols on Figure 2 object 311)

- Pump: 1
- Pressure release device: 2
- Line: 3
- Contact region: 4
- Sluice: 5
- Inlet with temperature sensor: 6
- Outlet with temperature sensor: 7
- Control means: 8
- Control and data line: 9
- Gas conveyor: 10
- Measurement point *T_{1,E}*, *p_{E}*, η*_{E}*: 11
- Measurement point *T_{2,CF}*: 12
- Measurement point *T_{1,CF}*: 13

## Claims

1. A process for the preparation of an ester, at least based on
a. at least one alcohol component,
b. at least one carboxylic acid component,
as process components, comprising the process steps:
i. provision of the process components,
ii. reaction of the process components to give an ester A,
iii. after-treatment of the ester A to give an ester in granule form,
wherein the after-treatment comprises at least a process for the preparation of an ester in granule form in a spray tower, with at least the following after-treatment steps:
aa. provision of the ester A as a fluid stream,
bb. optionally addition of auxiliary substances,
cc. charging of the fluid stream with pressure,
dd. release and division of the fluid stream via a pressure release device into a contact region to give a discontinuous fluid stream and
ee. cooling of the discontinuous fluid stream in the contact region by a cooling fluid in counter-current, to give a particle stream,
wherein, according to formula (I), the variable *I₁* is in a range of from 0.05 to 4.0, ${I}_{1} = \left|\frac{{p}_{E}}{{T}_{2 , CF} - {T}_{1 , CF}}\right|$
where
p_{E} = pressure of the fluid stream before the pressure release device;
T_{1,CF} = temperature of the cooling fluid on entry into the contact region;
T_{2,CF} = temperature of the cooling fluid on exit from the contact region;
and
wherein, according to formula (II), the variable *I₂* is in a range of from 0.04 to 3.0, ${I}_{2} = \left|\frac{{T}_{1 , E} - {T}_{m , E}}{{T}_{2 , CF} - {T}_{1 , CF}}\right|$
where
T_{m,E} = melting point of the ester A;
T_{1,E} = temperature of the ester A as the fluid stream before the pressure release device;
T_{1,CF} = temperature of the cooling fluid on entry into the contact region;
T_{2,CF} = temperature of the cooling fluid on exit from the contact region;
and
wherein, according to formula (III), the variable *I₃* is chosen in a range of from 0.04 to 0.95, ${I}_{3} = \frac{{p}_{E}}{{η}_{E}}$
where
p_{E} = pressure of the fluid stream before the pressure release device, determined in [bar];
η_{E} = viscosity of the fluid stream before the pressure release device, determined in [mPa·s].

2. A process for the preparation of an ester, at least based on
a. at least one alcohol component,
b. at least one carboxylic acid component,
as process components, comprising the process steps:
i. provision of the process components,
ii. reaction of the process components to give an ester A,
iii. after-treatment of the ester A to give an ester in granule form,
wherein the after-treatment comprises at least a process for the preparation of an ester in granule form in a spray tower and comprises at least the after-treatment steps:
aa. provision of the ester A as a fluid stream,
bb. optionally addition of auxiliary substances,
cc. charging of the fluid stream with pressure,
dd. release and division of the fluid stream via a pressure release device into a contact region to give a discontinuous fluid stream and
ee. cooling of the discontinuous fluid stream in the contact region by a cooling fluid in counter-current, to give a solid particle stream,
wherein, according to formula (I), the variable *I₁* is in a range of from 0.05 to 4.0, ${I}_{1} = \left|\frac{{p}_{E}}{{T}_{2 , CF} - {T}_{1 , CF}}\right|$
where
p_{E} = pressure of the fluid stream before the pressure release device;
T_{1,CF} = temperature of the cooling fluid on entry into the contact region;
T_{2,CF} = temperature of the cooling fluid on exit from the contact region;
or
wherein, according to formula (II), the variable *I₂* is in a range of from 0.04 to 3.0, ${I}_{2} = \left|\frac{{T}_{1 , E} - {T}_{m , E}}{{T}_{2 , CF} - {T}_{1 , CF}}\right|$
where
T_{m,E} = melting point of the ester A;
T_{1,E} = temperature of the ester A as the fluid stream before the pressure
release device;
T_{1,CF} = temperature of the cooling fluid on entry into the contact region;
T_{2,CF} = temperature of the cooling fluid on exit from the contact region;
or
wherein, according to formula (III), the variable *I₃* is chosen in a range of from 0.04 to 0.95, ${I}_{3} = \frac{{p}_{E}}{{η}_{E}} .$
where
p_{E} = pressure of the fluid stream before the pressure release device, determined in [bar];
η_{E} = viscosity of the fluid stream before the pressure release device, determined in [mPa·s],
or
a combination of two or more of these.

3. The process according to claim 1 or 2, wherein the particles obtainable as a particle stream in after-treatment step ee. have
- a mass mean particle size in the range of from 400 to 800 µm, and
- up to 20 wt.% of these particles have a particle size of less than 200 µm, and
- up to 20 wt.% of these particles have a particle size of more than 1,000 µm.

4. The process according to one of the preceding claims, wherein the particles obtainable as a particle stream in after-treatment step ee. have
- a mass mean particle size in the range of from 400 to 800 µm, and
- up to 15 wt.% of these particles have a particle size of less than 200 µm, and
- up to 15 wt.% of these particles have a particle size of more than 1,000 µm.

5. The process according to one of the preceding claims, wherein the entry temperature of the cooling fluid is in a range of from -5 to +30 °C.

6. The process according to one of the preceding claims, wherein the contact region is flowed through by the cooling fluid with a flow rate in a range of from 10,000 to 60,000 m³/h.

7. The process according to one of the preceding claims, wherein the fluid stream prior to atomization is charged with a pressure in a range of from 1,500 to 40,000 mbar.

8. The process according to one of the preceding claims, wherein the carboxylic acid component is chosen from the group consisting of caprylic acid, nonanoic acid, i-nonanoic acid, decanoic acid, i-decanoic acid, sebacic acid, palmitic acid, stearic acid, oleic acid, pelargonic acid, trimellitic acid, adipic acid, erucic acid, behenic acid, 12-hydroxystearic acid, HOOC-C₃₆H₇₂-COOH, phthalic anhydride, or a mixture of two or more of these.

9. The process according to one of the preceding claims, wherein the alcohol component is chosen from the group consisting of pentaerythritol, pentaerythritol dimer, n-octanol, i-tridecanol, 2-propylheptanol, behenyl alcohol, glycerol, trimethylolpropane, ethylene glycol, diethylene glycol, cetyl alcohol, stearyl alcohol or a mixture of two or more of these.

10. The process according to one of the preceding claims, wherein a catalyst which comprises one or more compounds chosen from the group consisting of tin oxalate, p-toluenesulphonic acid, sulphuric acid, hypophosphorous acid, lithium hydroxide, potassium hydroxide, magnesium hydroxide, calcium hydroxide and strontium hydroxide, tin oxide and tin or a mixture of two or more of these is employed as an additive.

11. The process according to one of the preceding claims, wherein the ester has between 1 and 5 ester groups.

12. A device comprising
a reaction region (110);
connected by fluid-conducting means to an after-treatment unit (311), comprising
- a pump (1) and
- at least one pressure release device (2),
wherein the pump (1) and the pressure release device (2) are connected by a fluid-conducting means (3),
- a contact region (4) following the pressure release device (2),
- an inlet for a cooling fluid (6) arranged in the contact region (4),
- an outlet for the cooling fluid (7) arranged above the inlet for the cooling fluid (6), and
- at least one control means (8).

13. A process for the preparation of an ester according to one of claims 1 to 11, wherein the device according to claim 12 is used.

14. A process for the preparation of a thermoplastic composition comprising the components
a1) a thermoplastic polymer,
b 1) a mould release agent, and
c1) optionally further additives,
comprising the process steps:
i) provision of a thermoplastic polymer or of a precursor of a thermoplastic polymer or both,
ii) provision of a mould release agent comprising an ester obtainable by a process according to one of claims 1 to 11,
iii) optionally provision of further additives,
iv) mixing of components i), ii) and optionally iii).

15. The process according to claim 14, wherein the mixing is carried out in accordance with at least one of the following measures:
M1) at or above the glass transition temperature of the thermoplastic polymer,
M2) wherein the mould release agent is more liquid than the thermoplastic polymer, or
M3) wherein at least a part of the mould release agent is added to the precursor of the thermoplastic polymer.

16. The process according to claim 14 or claim 15, wherein the thermoplastic polymer is based on polyesters or polyolefins to the extent of more than 90 wt.%.

17. The process according to one of claims 14 to 16, wherein components a1) to c1) are mixed with one another in relative amounts such that the thermoplastic composition obtained by mixing components a1) to c1) contains
a1) at least 60 wt.% of the thermoplastic polymer,
b1) 0.01 to 20 wt% of the mould release agent and
c1) 0 to 20 wt.% of the further additives,
in each case based on the total weight of the thermoplastic composition, the sum of components a1) to c1) being 100 wt.%.

18. A process for the production of a shaped article based on a thermoplastic composition, comprising the process steps:
I) provision of a thermoplastic composition obtainable according to one of claims 1 to 11,
II) heating of the thermoplastic composition to the glass transition temperature or to a temperature above the glass transition temperature of the thermoplastic polymer,
III) production of a shaped article from the heated thermoplastic composition prepared in process step II).

19. The process according to claim 18, wherein in a further process step IV) at least a part region of the shaped article obtained in process step III) is reduced in its mass cross-section compared with process step III).

20. The process according to claim 19, wherein the reduction in cross-section is carried out by applying a gas pressure.

21. The process according to one of claims 18 to 20, wherein the shaped article is chosen from a group consisting of: a container, a film, a fibre or at least two of these.

22. A process for the production of a packed product, comprising as process steps:
a3) provision of a shaped article, obtainable according to one of claims 18 to 21, and a product;
b3) at least partial surrounding of the product with the shaped article.

23. A process for the production of an at least partly coated object, comprising:
a4) provision of a coating composition comprising at least 10 wt.%, based on the coating composition, of an ester according to one of claims 1 to 11, or a thermoplastic composition according to one of claims 13 to 17, and a solid substrate;
b4) mixing of the coating composition and the substrate, the coating composition being at least partly liquid.

24. Further processing product comprising an ester which can be prepared by a process according to one of claims 1 to 11, as an additive, and at least one functional component chosen from the group consisting of thermoplastic polymer, enzyme, curing agent of an adhesive, paraffin, oil, colouring agent, hair or skin care substance, polymer dispersion, lime mud, lubricant or emulsifier, or a combination of two or more of these.

25. Use of an ester obtainable by a process according to one of claims 1 to 11 as an additive in a composition which is chosen from the group consisting of thermoplastic composition, shaped article, detergent, adhesive, defoamer, lubricant formulation, lacquer, paint, cosmetic formulation, soil compacting agent, drilling mud, hydraulic oil or dispersion.
